# EUROPEAN PATENT SPECIFICATION

(11) **EP 3 583 918 B1**
(45) Date of publication and mention of the grant of the patent: **26.06.2024**
(21) Application number: 19173074.6
(22) Date of filing: 11.01.2012
(51) Int. Cl.: A61F 2/24, A61F 2/95

(54) **SYSTEM FOR RETROGRADE PERICARDIAL RELEASE OF A PROSTHETIC HEART VALVE**
SYSTEM ZUR RETROGRADEN PERIKARDIALEN FREISETZUNG EINER HERZKLAPPENPROTHESE
SYSTÈME POUR UNE LIBÉRATION PÉRICARDIQUE RÉTROGRADE D'UNE VALVULE CARDIAQUE PROTHÉTIQUE

(30) Priority: 11.01.2011 US 201161431710 P; 11.01.2011 EP 11150640
(43) Date of publication of application: 25.12.2019
(62) Divisional of application: 12703462.7
(73) Proprietor: Boston Scientific Medical Device Limited, Galway (IE)
(72) Inventor: Essinger, Jacques, 8832 Wollerau (CH); Delaloye, Stephane, 8180 Bülach (CH); Hefti, Jean-Luc, 1400 Cheseaux-Noréaz (CH); Biadillah, Youssef, 75014 Paris (FR); Mantanus, Luc, 1007 Lausanne (CH); Mallabiabarrena, Iker, 1217 Meyrin (CH); Passerini, Reynald, 1006 Lausanne (CH); Paris, Michael, San Francisco, CA 94114 (US); Lombardi, Fabien, 1008 Prilly (CH)
(74) Representative: Peterreins Schley

(56) References cited:
- US-A- 5 445 646
- US-A1- 2006 058 866
- US-B1- 6 254 628

## Description

### BACKGROUND OF THE DISCLOSURE

Conventional approaches for cardiac valve replacement require the cutting of a relatively large opening in a patient's sternum ("sternotomy") or thoracic cavity ("thoracotomy") in order to allow the surgeon to access the patient's heart. Additionally, these approaches require arresting of the patient's heart and a cardiopulmonary bypass (i.e., use of a heart-lung bypass machine to oxygenate and circulate the patient's blood). In recent years, efforts have been made to establish a less invasive cardiac valve replacement procedure, by delivering and implanting a cardiac replacement valve via a catheter percutaneously (i.e., through the skin) via either a transvascular approach - delivering the new valve through the femoral artery, or by a transapical route, where the replacement valve is delivered via a catheter (or the like) between ribs and directly through the wall of the heart to the implantation site.

While less invasive and arguably less complicated, percutaneous heart valve replacement therapies (PHVT) still have various shortcomings, including the inability for a surgeon to ensure proper positioning and stability of the replacement valve within the patient's body. Specifically, if the replacement valve is not placed in the proper position relative to the implantation site, it can lead to poor functioning of the valve. For example, in an aortic valve replacement, if the replacement valve is placed too high, it can lead to valve regurgitation, instability, valve prolapse and/or coronary occlusion. If the valve is placed too low, it can also lead to valve regurgitation and mitral valve interaction.

The foregoing description of related art is not intended in any way as an admission that any of the documents described therein, including issued U.S. patents and pending U.S. patent applications, are prior art to embodiments according to the present disclosure. Moreover, the description herein of any disadvantages associated with the described systems, methods and devices, is not intended to limit inventions disclosed herein. Indeed, aspects of the disclosed embodiments may include certain features of the described systems, methods, and devices without suffering from any described disadvantages.

US 2006/58866 A1 discloses a deployment system for a self-expanding endoluminal device.

US 5 445 646 A discloses a single layer hydraulic sheath stent delivery apparatus.

US 6 254 628 B1 discloses a stent and stent catheter for intracranial use.

### SUMMARY OF THE DISCLOSURE

The present invention relates to a cardiac valved stent delivery system for delivering a cardiac valved stent to the heart, comprising a cardiac valved stent and a cardiac valved stent delivery device, wherein the delivery device comprises a tip and the tip comprises a tip sheath and a hydraulic actuator piston component which is coupled to the tip sheath, wherein the delivery device is configured to use hydraulic action which is transmitted to the hydraulic actuator piston component of the tip of the delivery device via a fluid-tight conduit to apply driving force to the hydraulic actuator piston component which distally displaces the tip sheath, wherein the tip comprises a rear sheath having a stepped shape that is slidably retractable with regards to the piston.

Further embodiments of the invention are recited in the dependent claims.

### BRIEF DESCRIPTION OF THE DRAWINGS

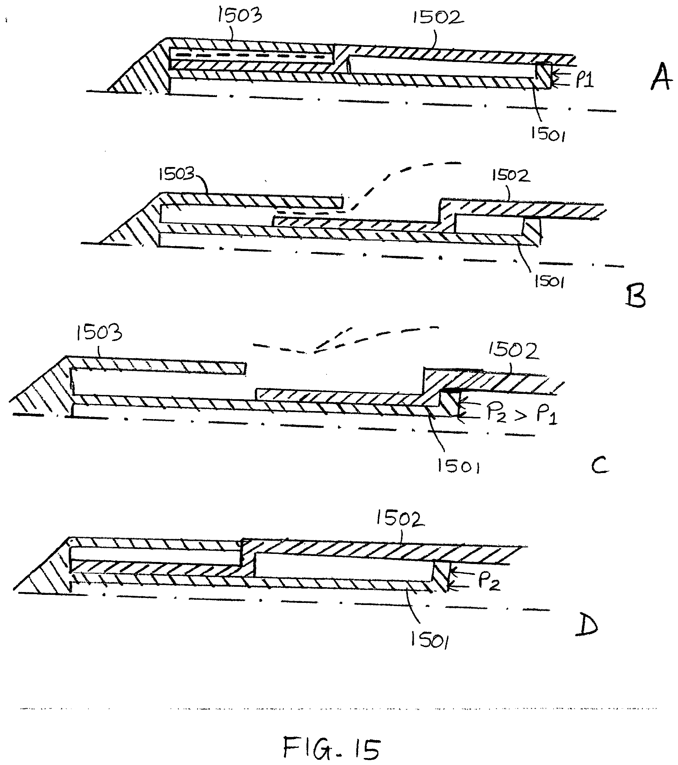

For a better understanding of the embodiments of the present disclosure, reference is made to the following description, taken in conjunction with the accompanying drawings, in which like reference characters refer to like parts throughout. The embodiment shown in Fig. 15 belongs to the claimed invention. Other figures are disclosed for illustrative purposes but may contain features which may optionally be implemented in embodiments of the claimed invention.
FIG. 1 is a schematic showing a split sheath stent delivery device according to some embodiments of the disclosure.
FIG. 2 is a schematic showing a split sheath stent delivery device with a tip sheath that can be extended from the proximal end of the distal tip of the device, proximally over a stent in order to recapture the stent according to some embodiments of the disclosure.
FIG. 3 is a schematic showing a split sheath stent delivery device with telescoping sheaths according to some embodiments of the disclosure.
FIGS. 4A and 4B are schematics showing a rolling membrane stent delivery device according to some embodiments of the disclosure, wherein FIG. 4A shows the rolling membrane covering the stent and FIG. 4B shows the rolling membrane being removed from the stent so that it can be deployed.
FIG. 5. is a schematic showing a rolling membrane stent delivery device with an outer sheath covering the proximal end of the stent according to some embodiments of the disclosure.
FIG. 6 is a schematic showing a rolling membrane stent delivery device with an outer sheath covering the proximal end of the stent according to some embodiments of the disclosure.
FIG. 7 is a schematic showing a stent delivery device that is actuated by a vacuum according to some embodiments of the disclosure.
FIG. 8 is a schematic showing a stent delivery device wherein a membrane covers the stent and is removed from the stent through pulling a pull wire through a central catheter according to some embodiments of the disclosure.
FIG. 9 is a schematic showing a stent delivery device wherein a membrane covers the stent and is removed from the stent through pulling two pull wires through a central catheter according to some embodiments of the disclosure.
FIG. 10 is a schematic showing a castellated stent holding device according to some embodiments of the disclosure. The stent has elongations on its distal end that match with the tabs when the holding device engages the stent and match the indentations when the holding device releases the stent.
FIG. 11 is a schematic showing a stent delivery device with a distal tip that opens with an umbrella-like motion according to some embodiments of the disclosure.
FIG. 12 is a schematic showing a stent delivery device with a distal tip that opens with an umbrella-like motion that is closed open by a balloon arranged on the outside of the distal tip according to some embodiments of the disclosure.
FIG. 13 is a schematic showing a stent delivery device wherein the distal end of the stent is held by a wire according to some embodiments of the disclosure.
FIG. 14 is a schematic showing a stent delivery device wherein the proximal end of the stent is held by a wire according to some embodiments of the disclosure.
FIG. 15 is a schematic showing a stent delivery device that is powered by a fluid pressure system according to some embodiments of the disclosure.
FIG. 16 shows the placement of a double polyester (PET) fabric skirt 1603 relative to a stent component 1601, as well as placement of a valve-component within the stent 1602 according to some embodiments of the disclosure.
FIGS. 17A to 17E show the size and shape of the elements of the stent component in the expanded and non-expanded configuration according to some embodiments of the disclosure.
Fig. 18 is a schematic illustration of a stent-valve delivery system.
Fig. 19 is a schematic section through a portion of a subassembly usable in the stent-valve delivery system of Fig. 18. Fig. 20 is a schematic section similar to Fig. 19, showing a first example modification of the segment profiles.
Fig. 21 is a schematic section similar to Figs. 18 and 19, showing a second example modification of the segment profiles.
Fig. 22 is a schematic illustration illustrating a first example of distal portion of the delivery system of Fig. 18.
Fig. 23 is a schematic illustration illustrating a second example of distal portion of the delivery system of Fig. 18.
Fig. 24 is a schematic illustration illustrating a third example of distal portion of the delivery system of Fig. 18.

### DETAILED DESCRIPTION

Embodiments of the present disclosure are directed to systems, methods, and devices for cardiac valve replacement. For example, such methods, systems, and devices may be applicable to the full range of cardiac-valve therapies including, for example, replacement of failed aortic, mitral, tricuspid, and pulmonary valves. Some embodiments may facilitate a surgical approach on a beating heart without the need for an open-chest cavity and heart-lung bypass. Such minimally-invasive surgical approaches may reduce the risks associated with replacing a failed native valve in the first instance, as well as the risks associated with secondary or subsequent surgeries to replace failed artificial (e.g., biological or synthetic) valves.

### Stents, Valved-Stents

The present disclosure relates to systems for the implantation of stents and valved-stents. Valved-stents (which may also be referred to as "valved-stents"), according to some embodiments of the present disclosure, may include a valve component and at least one stent component (e.g., a single-valved-stent or a double-valved-stent ). The term "valve component" may refer generally to any suitable valve, whether an integral unit or formed by plural parts not forming an integral unit. The valve component may include a biological valve (e.g., porcine or bovine harvested valve), a synthetic valve (e.g., synthetic valve leaf- let made of biological tissue (e.g., pericardium), and/or synthetic valve leaflet material and/or a mechanical valve assembly) , and any other suitable material (s). The stent and valve components, according to some embodiments, may be configured for at least two configurations: a collapsed or contracted configuration (e.g., during delivery) and an expanded configuration (e.g., after implantation).

According to some embodiments, the valved-stents of the present disclosure may be used as replacement heart valves and may be used in methods for replacing diseased or damaged heart valves. Heart valves are passive structures that simply open and close in response to differential pressures on either side of the particular valve, and comprise moveable "leaflets" that open and close in response to such differential pressures. For example, the mitral valve has two leaflets and the tricuspid, aortic and pulmonary valves have three. The aortic and pulmonary valves are also referred to as "semilunar valves" due to the unique appearance of their leaflets or "cusps" and are shaped somewhat like a half-moon. The aortic and pulmonary valves each have three cusps.

The valve component may be designed to be flexible, compressible, host-compatible, and non-thrombogenic (for example). As previously noted, the valve component can be made from various materials, including, for example tissue that may be fresh, cryopreserved or glutaraldehyde fixed allografts or xenografts. Synthetic biocompatible materials such as polytetrafluoroethylene, polyester, polyurethane, nitinol or other alloy/metal foil sheet material and the like may also be used. However, the preferred material for the valve component is mammal pericardium tissue, particularly juvenile-age animal pericardium tissue.

Replacement heart valves are generally categorized into one of three categories: artificial mechanical valves, transplanted valves, and tissue valves. Mechanical valves are typically constructed from non-biological materials such as plastics, metals, and other artificial materials. Transplanted valves are natural valves taken from cadavers, which are typically removed and frozen in liquid nitrogen, and are stored for later use. Such "frozen" valves are typically fixed in glutaraldehyde to eliminate antigenicity. Artificial tissue valves are valves constructed from animal tissue, such as bovine or porcine tissue. Efforts have also been made at using tissue from the patient for which the valve will be constructed. Such regenerative valves may also be used in combination with the stent components described herein. The choice of which type of replacement heart valves are generally based on the following considerations: hemodynamic performance, thrombogenicity, durability, and ease of surgical implantation.

In some embodiments, tissue valves constructed by sewing the leaflets of pig aortic valves to a stent to hold the leaflets in proper position, or by constructing valve leaflets from the pericardial sac of cows or pigs and sewing them to a stent, are utilized. See e.g., U.S. Patent Publication No. 2005/0113910. Methods of creating artificial tissue valves is described in U.S. Patent Nos. 5,163,955, 5,571,174, and 5,653,749.

According to some embodiments, the valve component is attached to the inner channel (also referred to as lumen) of the stent member. This may be accomplished using any means known in the art. For example, the valve component may be attached to the inner channel of the stent member by suture or stitch, for example, by suturing the outer surface of the valve component pericardium material to the stent member, and for example, attaching the valve component to the commissural posts 2 of the stent member. The attachment position of the valve may be closer to the proximal end of the stent chosen with the understanding that the annulus of the native valve being replaced will preferably engage the outer surface of the stent at the groove by the upper anchoring crown 3.

According to some embodiments, the stent component comprises opposite end regions with an intermediate portion therebetween. The intermediate portion may comprise a crown and our one or more (e.g. radial) projections intermediate the opposite end regions.

According to some embodiments, the stent component comprises a ventricular end and/or portion intended to be positioned in use at or towards the ventricle of the heart, and an aortic end and/or portion intended to be positioned in use at or towards the aorta. The configurations of the ventricular and aortic portions of the stent component may be different. For example, the ventricular portion may comprise a crown (e.g. having a conical portion) that diverges towards the ventricular end.

Also for example, the aortic portion may comprise a crown (e.g. having a conical portion) that diverges towards the aortic end. Additionally or alternatively, the aortic portion may comprise one or more arches. Optionally the apexes of the arches are positioned at or towards the aortic end of the stent component. The stent component may be configured such that the aortic portion should be deployed before the ventricular portion. In some examples, the aortic portion deployment of the aortic portion may be substantially completed before deployment of the ventricular portion commences. The aortic end of the stent component may optionally be the first region of the stent to be deployed, or another region of the aortic portion may be deployed before the aortic end.

### Cardiac Valved-stent Delivery System

The present disclosure provides a delivery system for delivering the valved-stents. For example, some embodiments provide a cardiac valved-stent delivery system that includes an inner assembly and an outer assembly. The inner assembly may include a guide wire lumen (e.g., polymeric tubing), a conduit for a pull wire or for transmission of vacuum, pneumatic or hydraulic force, and a stent holder for removable attachment to a valved-stent. The outer assembly may include a sheath and/or a membrane and/or one or more wires. The inner member and the outer member may be nested, e.g. co-axially positioned, and slidable, releasable or rollable relative to one another in order to transition from a closed position to an open position, such that in the closed position the sheath and/or membrane encompasses and/or one or more wires hold the valved- stent attached to the stent holder and thus constrains expansion of the valved-stent. In the open position, the outer sheath and/or membrane encompasses and/or one or more wires may not constrain expansion of the valved-stent and thus the valved- stent may detach from the stent holder and expand to a fully expanded configuration.

Some embodiments provide a cardiac valved-stent delivery system that includes plural assemblies nested one within at least another. The delivery system may include a portion configured for deploying and/or restraining until a time of deployment, a ventricular portion of the stent-valve. The delivery system may include a portion configured for deploying and/or restraining until a time of deployment, an aortic portion of the stent. The delivery system may a stent-holder portion configured for interengagement with the stent-valve to positively retain the stent- valve in a predefined axial position with respect to the delivery device. The above portions may optionally be defined by at least two, and optionally at least three, distinct assemblies. Figure15 illustrates an embodiment of the claimed delivery device. The delivery system, according to some embodiments, allows for a minimally-invasive surgical approach whereby valve replacement surgery is performed on a beating heart without the need for an open-chest cavity and heart-lung bypass. The heart can be penetrated trans-apically through a relatively small opening in the patient's chest (e.g., an intercostal space - a region between two ribs). From this access point, the left ventricle is penetrated at the apex of the heart. The surgery can also be performed transvascularly, for example, access via the femoral artery ( transfemoral access) and/or access via the subclavian artery ( transsubclavian or transaxilliary access) and/or access via the ascending aorta (direct aortic access).

The delivery device may be used to position and release the aortic bioprosthesis or stented replacement valve at the intended location over the patient's native, aortic valve via transapical or transvascular access.

When deployed, the valved-stent can be automatically detached from the stent holder via, for example, the self-expandable properties of the stent, thereby leaving the upper and lower crown fully expanding over the native leaflets respectively within the left ventricle outflow tract. Careful withdrawal of the delivery system tip can be performed through the fully deployed and functional bioprosthesis under fluoroscopic control to avoid any valve dislodgement (for example).

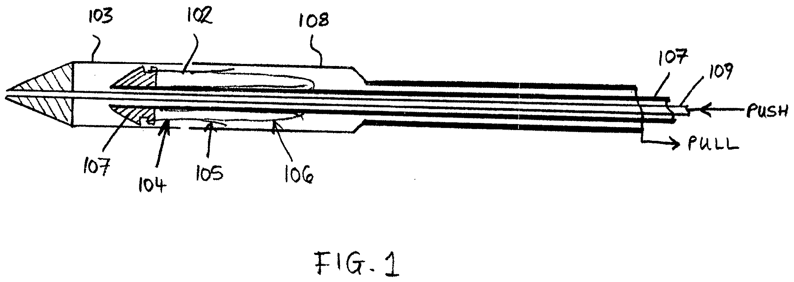

Different embodiments of the delivery devices described herein cover and hold different portions of a collapsible/expandable valved-stent. For example, the delivery device, shown in Figure 1, is configured to minimize the extent of penetration into the ventricle. The stent may be held in place by split sheaths: the tip or distal sheath 103 and the proximal sheath 108. In some embodiments, in order to minimize ventricle penetration, the portion of the tip sheath 103 distal to the stent 102 should be as small as possible. The tip sheath may cover the entire distal portion of the stent 104, or it could cover merely a sufficient length to ensure that the distal portion does not deploy in use and will not spring from the stent holder 107. The distal portion may be the ventricular portion of the stent. The ventricular portion may include a crown (lower crown). The tip sheath may also extend further proximally, if desired, so as to cover a part or all of the upper crown 105 and hence prevent deployment of the upper crown before the stabilization arches 106.

Sheath parts 103 and 108 may meet by abutting edge-to-edge, overlap, or may overlap such that a portion of one sheath may fit (male-female) within a portion of the other. For example, one sheath may have a slightly raised lip or rim to define a female mouth for receiving the edge of the other sheath.

The twin sheaths may be manipulated by pushing/pulling on nested (e.g. coaxial or concentric) control tubes, with respect to the stent-holder mounting tube 107. In addition, for example, an outer catheter connected to the proximal sheath 108 may be pulled proximally to reveal and/or release the proximal end of the stent. Subsequently, an inner catheter 109 may be pushed distally to push the distal sheath distally away from the distal end of the stent, thus revealing and/or releasing it (according to some embodiments).

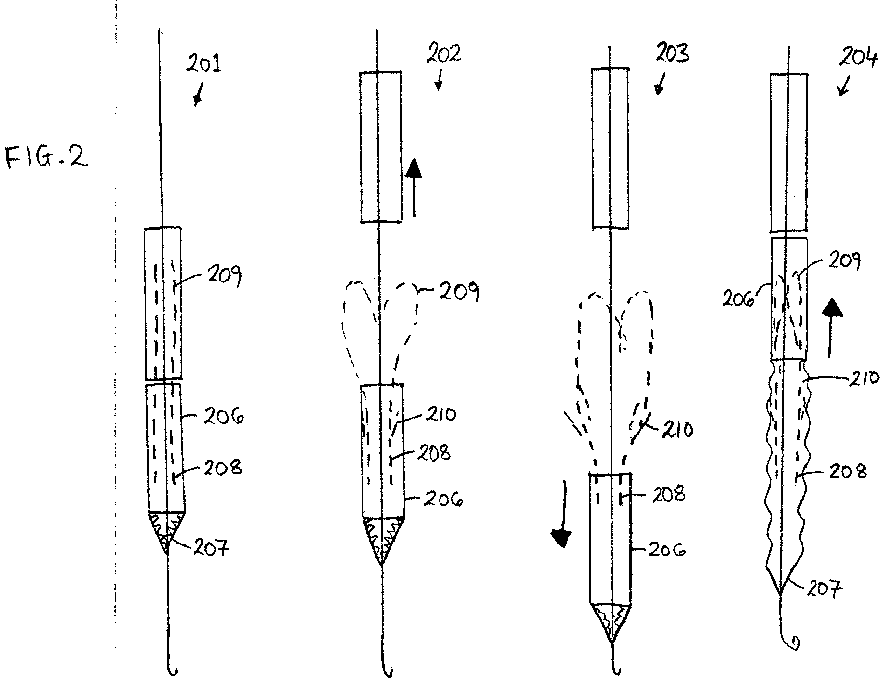

In other examples, which may be a variation of the design shown in Figure 1, and as shown in Figure 2, a device with a membrane extending proximally from the distal tip of the device may be used in facilitating recapture of the stent. In such embodiments, recapture is facilitated until, for example, the release of the lower crown 208. In normal use, the tip component may be coupled with the tip sheath as one unit, as shown at 201, 202 and 203, where 201 is in reference to the stent sheathed, 202 is in reference to the deployment of the stabilization arches 209, 203 is in reference to the deployment of the upper crown 210 of the stent, and 204 is in reference to recapture of the deployed portions of the stent. It is worth noting that the tip component, in some embodiments, is able to be uncoupled from the tip sheath, as shown at 204. A tubular membrane of flexible, but substantially non-stretching material, and normally tightly collapsed into a compact form inside or just behind the tip component, is distensible between the tip component and the tip sheath 206.

To recapture after partial release of the stent, the tip sheath 206 is uncoupled from the tip component 207, and then drawn proximally (towards the aorta) to cover the upper crown 210 and stabilization arches 209, as shown at 204. The tip component preferably remains at its distal position, and the tubular membrane distends. Alternatively, to recapture the stent after partial release, the tip sheath 206 is uncoupled from the tip component 207, and the tube carrying the stent/stent-holder is pushed distally until it contacts the tip component. This relative movement drives the stent further into the distal sheath. Upon contacting the tip component, the tip component begins also to displace distally under the pushing force, while the distal sheath remains stationary under the influence of friction with the native anatomy. The displacement of the tip component relative to the tip sheath distends the tubular membrane. In some embodiments, the tubular membrane keeps the lower crown (and later possibly the upper crown) covered and restrained in collapsed form. Suitable control wires or tubes (and/or other controls) may be used to control the tip component and the tip sheath. A releasable coupling (e.g. bayonet) may be used to initially (for example) couple the tip component to the tip sheath.

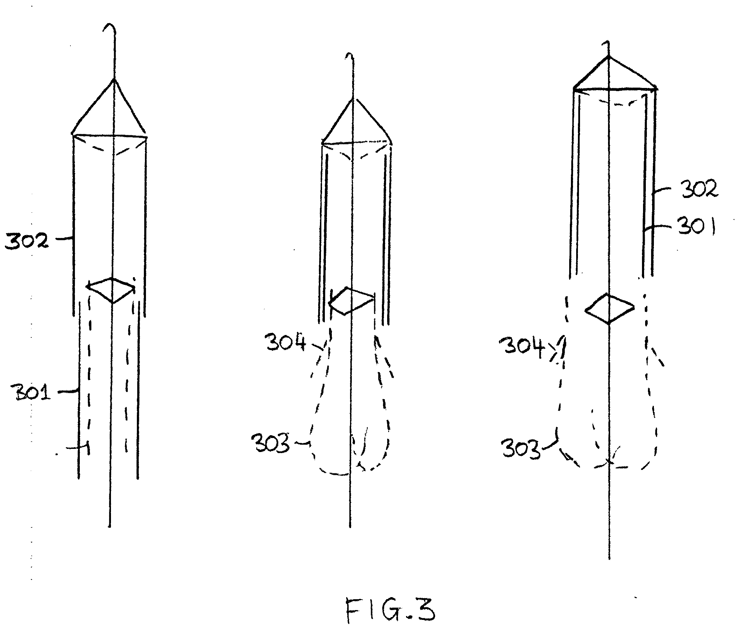

Figure 3 illustrates an example which is similar to that of Figure 1, except that, instead of the proximal sheath 301 being retracted towards the handle, it is advanced into or over the tip sheath 302, defining a telescoping arrangement, for example. Although such a system may not minimize penetration into the ventricle, as compared to the design of Figure 1, it can nevertheless achieve a modest to significant reduction (e.g., between about 10% to about 75%, and more preferably, near 50% reduction, for example) as compared to advancing a full-length single sheath into the ventricle. In some embodiments, the system/device of Figure 3 may also achieve release of the stabilization arches 303 before the upper crown 304.

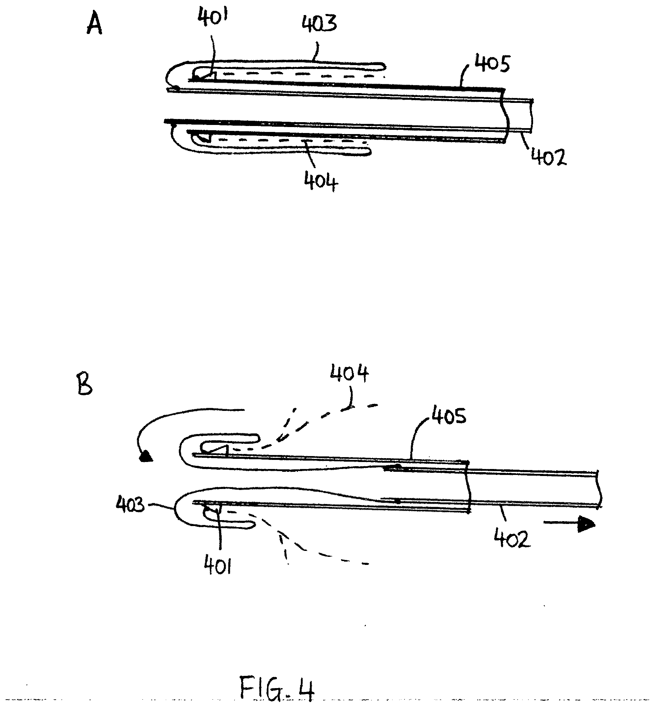

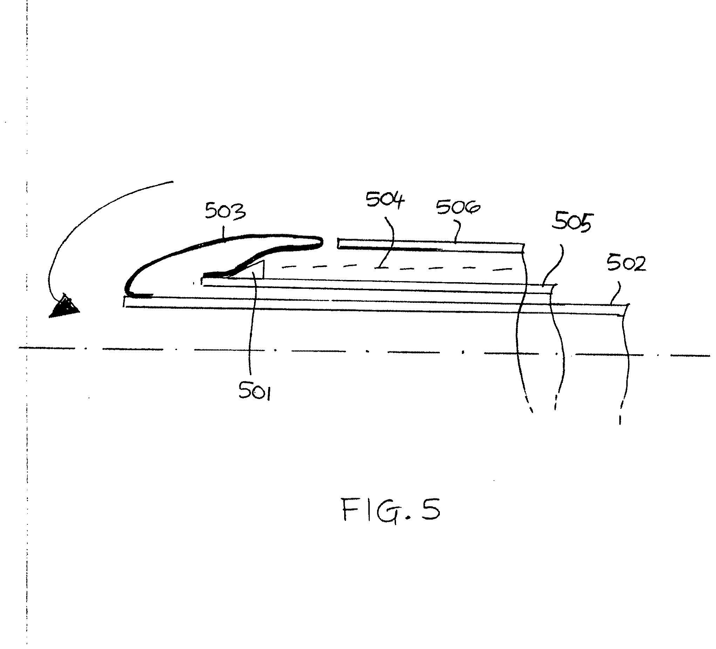

Figures 4 and 5 show a stent delivery system with an everted or rolling membrane 403 or 503 defining a folded (e.g., dual wall) cuff over at least a portion of the stent 404 or 504. The "inner" wall that contacts the stent is fixed to the stent holder 401 or 501 on an outer tube 405 or 505. The "outer" wall extends distally towards the tip, where it is attached to a puller tube 402 or 502. Upon pulling of the tube proximally, the membrane material de-everts from its proximal end, and rolls inside the outer tube through the distal tip of the device. The portion of the stent covered by the membrane is exposed progressively from its proximal end.

The membrane material is pulled into the outer tube through the distal tip, so that release does not require any additional penetration of the device into the ventricle. There is also no direct sliding of the membrane against the stent surface - instead only a rolling motion. This reduces friction, permitting the membrane to be relatively tight. The membrane may be lubricated to facilitate sliding movement over itself and into the distal end of the tube.

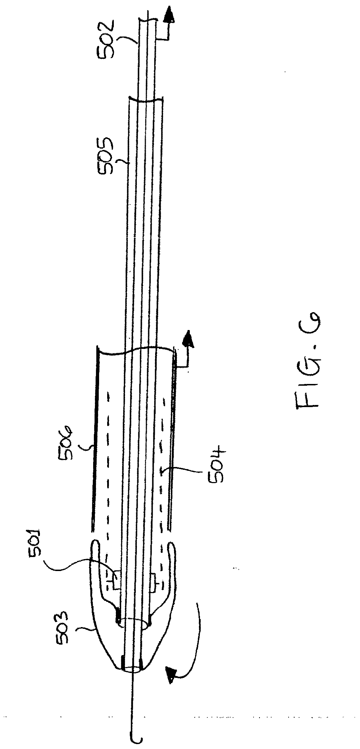

The version shown in Figure 4 presents a full-length rolling membrane that covers substantially the entire length of the stent. Such an arrangement could be used to release the stabilization arches first, before the upper crown, followed by the lower crown of a stent. The version shown in Figure 5 combines a shorter membrane with a displaceable rear or proximal sheath 506 like the one shown in Figure 1. Using a shorter membrane may reduce any risk of the membrane becoming trapped during the rolling manipulation, or as the material is drawn into the distal end of the tube. A more expanded view is shown in Figure 6.

In either of Figs. 4 and 5, instead of drawing the rolling membrane into the distal end of the tube by retracting the puller tube 402 or 502, it also envisaged to further extend the tube 402 or 502 distally. In such case, the tube 402 or 502 may act like a "pusher" or "extender", such that the membrane extends distally with distal extension movement of the tube 402 or 502, instead of being drawn proximally into the delivery system.

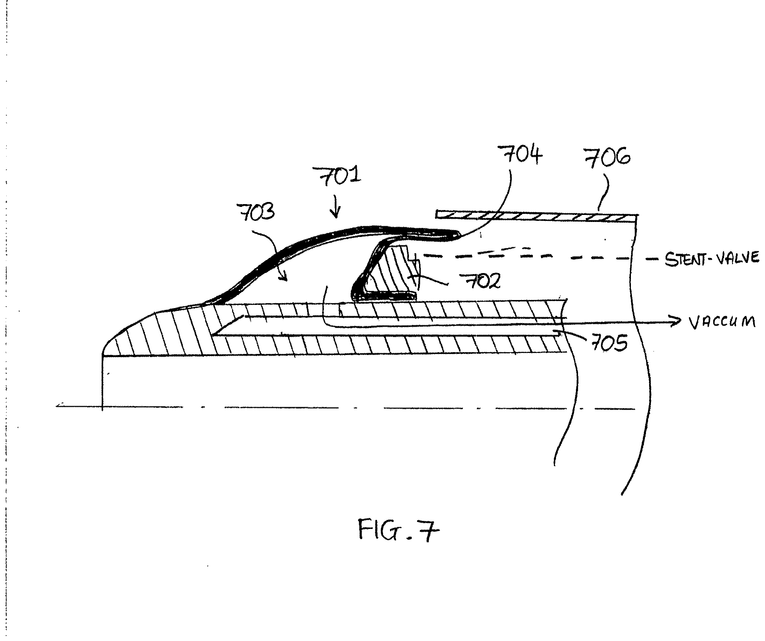

Figure 7 shows a stent delivery device with a short sheath 701 at the distal tip, for holding the lower crown 702. A retracting rear sheath 706, as shown in Figure 1, would be used to cover/release the remainder of the stent.

The distal sheath comprises a balloon-cuff with a hollow pocket 703 from which projects with a tubular wing or flap portion 704 of the cuff for overlapping the lower crown. The interior of the hollow pocket communicates with a suction conduit 705. Upon applying sufficient suction, the cuff is pulled against the surface of the tube, restraining the lower crown against expansion. Upon releasing the suction and/or applying positive pressure, the cuff lifts and stands slightly distally with regard to its suction-collapsed position, such that the tubular wing no longer overlaps the lower crown significantly. Under positive pressure, the shape of the cuff could further change to lift or move the flap distally of the lower crown. Positive pressure could also elongate the delivery device tube, further displacing the cuff distally.

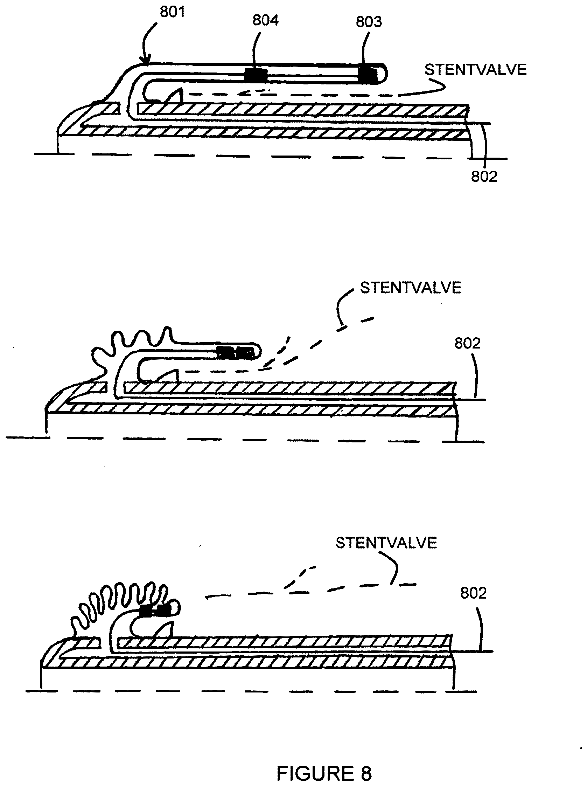

Figure 8 shows a sheath made of flexible membrane material 801, and collapsible by pulling on a pull wire 802 that extends from the membrane towards the tip, and then through the length of the delivery catheter to the handle at the proximal end. The pull wire is attached to an annular ring 803 supporting the membrane, which also serves as a marker that can be observed using suitable equipment (e.g. RF). As second marker ring 804 defines the initial stop position, which corresponds to the stabilization arches and upper crown having been released, just prior to commencing unsheathing of the lower crown.

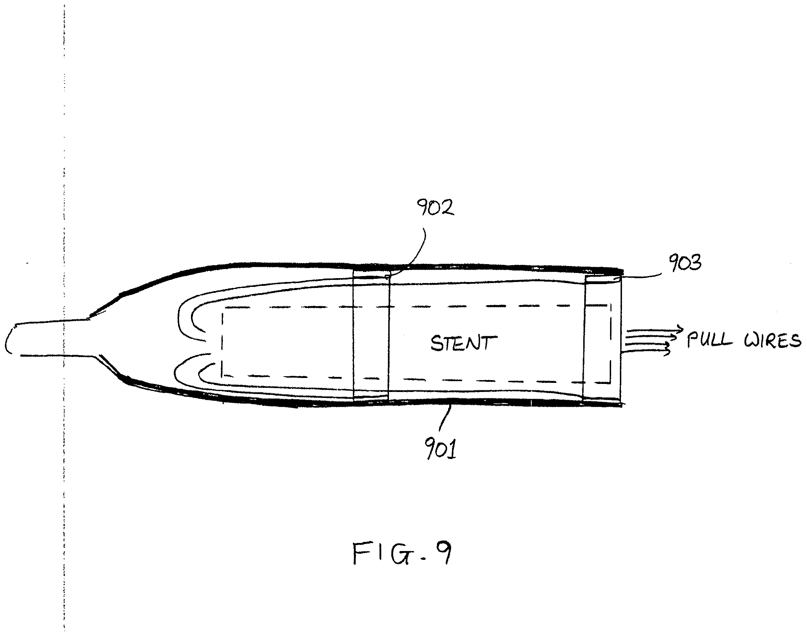

In Figure 8, a single pull wire is used attached to the proximal marker ring 803. The middle marker ring 804 (initial stop position) is a passive ring that begins to move only when the proximal ring 803 begins to bear against it. In Figure 9, a second pull wire 901 is attached to the middle ring 902 to control its movement. The user would pull on pull wire 1 to move the proximal ring 903 to uncover the stabilization arches and upper crown, followed by pulling on pull wire 2 or both 1 and 2 to move the middle ring 902 to uncover the lower crown.

Although a full length sheath is illustrated, the pull-wire sheath can also cover only the lower crown at the distal end. A second sheath that moves proximally could cover the remainder of the stent (upper crown and stabilization arches). This second sheath could be another pull-wire collapsible sheath, or it could be a sliding sheath (e.g. the rear or proximal sheath shown in Figure 1).

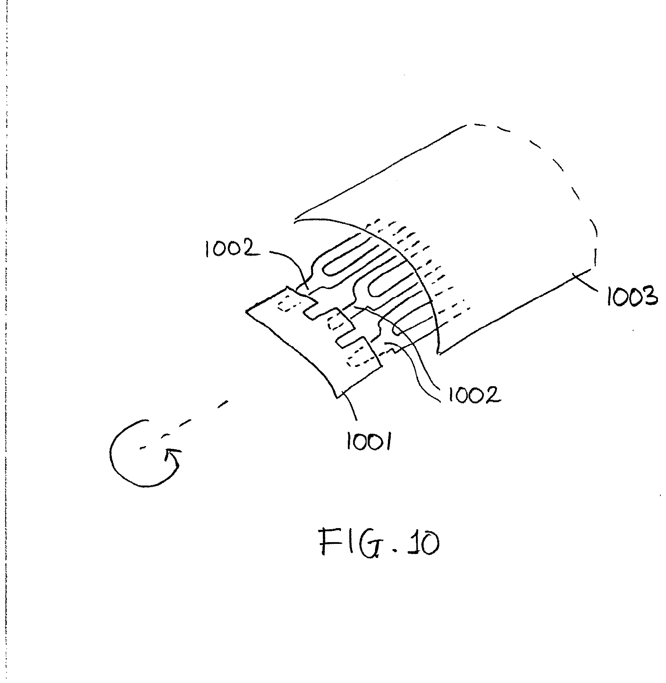

Figure 10 shows a stent delivery device including a "rear or proximal sheath" 1003 like the one shown in Figure 1, coupled with a castellated cover 1001 for the distal end of the lower crown of the stent. The lower crown may optionally have special elongations as attachment elements 1002. The castellated cover may be the stent holder of the delivery catheter itself.

The cover restrains the lower crown against expansion. When release of the lower crown is desired, the cover/stent holder is rotated, so that the clearances between the castellations align with the attachment elements. The attachment elements can pass through the clearances allowing the lower crown to expand. The castellations/clearances need not be rectangular; any suitable shape could be used. The number of clearances may be the same as the number of attachment elements or greater.

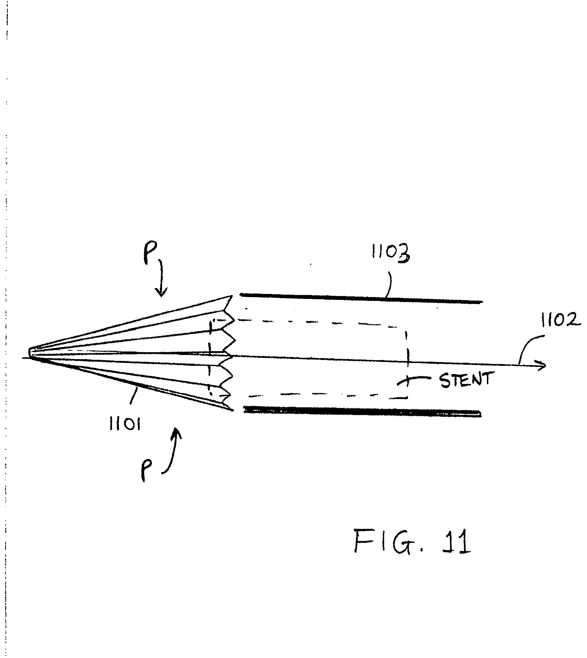

Figure 11 shows a stent delivery device with a catheter tip 1101 capable of changing configuration. In the specific design, the catheter tip is expandable, like an umbrella. The tip construction may itself be umbrella-like. It may have pleats or simply comprise elastically stretchable material. The tip may comprise a skeletal structure carrying a web, or it may be generally homogeneous.

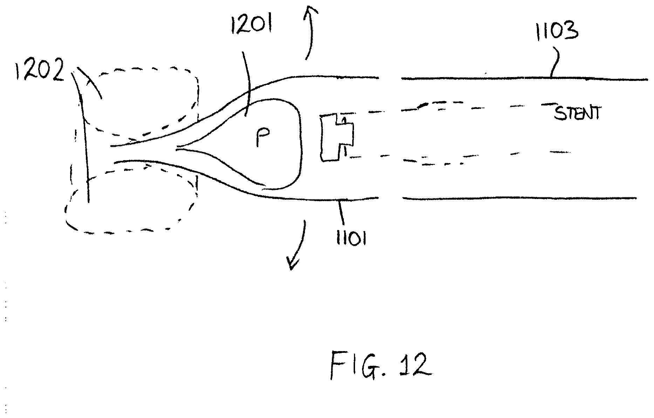

The tip may be self-biased closed. In certain situations this is a better failsafe mode so that the catheter can be withdrawn in its closed state should problems occur. Pulling on a pull- wire 1102, or inflating an internal balloon 1201 as shown in Figure 12 may expand the tip.

Alternatively, the tip may be self-biased open. Maintaining tension on a pull-wire may retain the tip closed until opening is desired (release tension to allow opening). Alternatively, applying suction to an internal chamber 1201 as shown in Figure 12 or applying a "closing" pressure by means of an external torroid ballon 1202 may retain the tip closed until opening is desired.

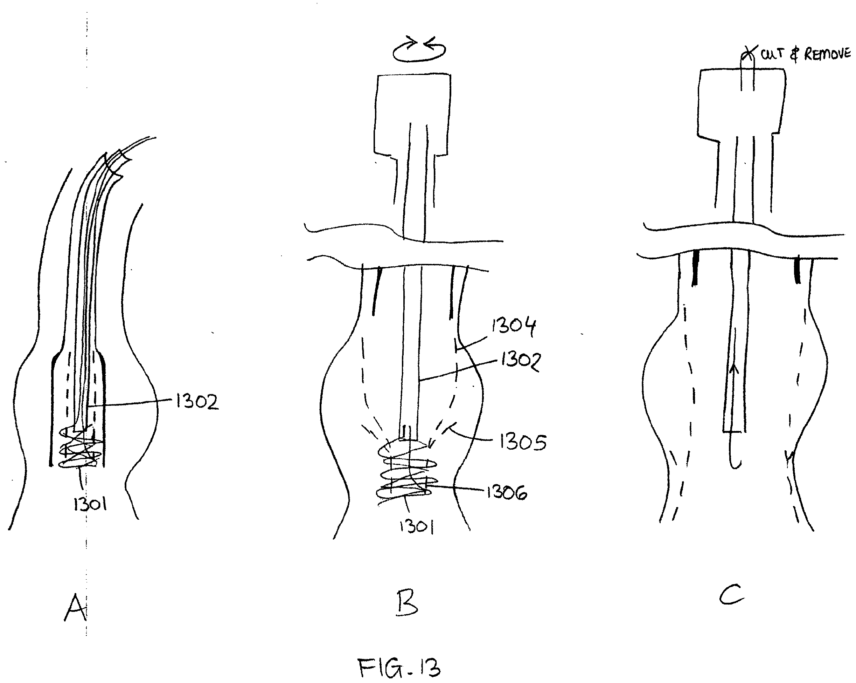

Figure 13 shows a stent delivery device configured to envelop the lower crown in a net or cage made from one or more continuous filaments of elongate suture wire 1301. Tensioning the filaments compresses the lower crown, and maintaining tension keeps the lower crown in its compressed non-deployed state, as shown in Figure 13A. A central lumen 1302 within the catheter provides leverage support for the filaments. The filament extends through the catheter to the proximal handle. The upper crown and stabilization arches are restrained by a separate sheath 1303 (e.g. as shown in Figure 1).

In use, the rear sheath is first displaced proximally, as shown in Figure 13B to release the stabilization arches 1304 and upper crown 1305. Thereafter, the lower crown 1306 is deployed progressively by relaxing the tension in the filament (s) 1301 progressively via the proximal handle, as shown in Figure 13C. If desired, the lower crown can be re-collapsed by again tightening the filament (s). Once the lower crown is expanded to a desired deployed state, the filament (s) is/are cut at the proximal handle, and withdrawn to disengage completely from the stent.

The upper crown can also be restrained by the filament(s). The upper crown could be restrained by the same filaments as the lower crown, so that positioning and seating of the stent is a progressive operation, in which both upper and lower crowns are deployed progressively but at the same time. Alternatively, different filaments could be provided for restraining the upper and lower crowns, each individually controllable to release the respective crown independently of the other. In a similar manner, the stabilization loops could also be restrained by filament.

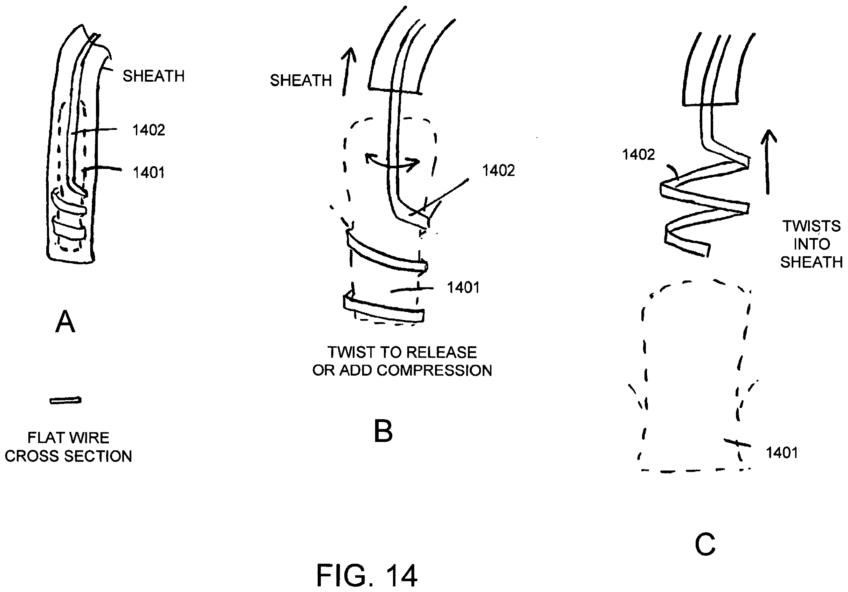

Figure 14 shows a stent delivery device with a self-supporting shaped flat wire or ribbon 1401, that has a helical thread shape. The ribbon envelopes at least the lower crown, and optionally the upper crown. The stabilization arches are restrained by a solid sheath 1402 (e.g. the rear sheath shown in Figure 1).

Figure 14B shows a state of partial release, in which the sheath is retracted, releasing the stabilization arches. The crowns are released by relative rotation of the ribbon. Rotation in one direction "unscrews" the stent from the ribbon. Rotation in the other direction "recompresses" or further compresses the crown(s).

After ultimate release, as shown in Figure 14C, the ribbon is twisted into the mouth of the retracted sheath, allowing the tip assembly to be withdrawn.

Figure 15 shows an embodiment of the claimed invention, specifically a stent delivery device that uses fluid action, transmitted to the delivery device tip via a fluid-tight conduit, to apply some driving or restraining force, instead of using a pullwire. The fluid can be a liquid (hydraulic), such as saline, or a gas (pneumatic). The idea of fluid action could be substituted in any of the preceding embodiments where appropriate to replace pull wire action.

The drawings show one example of a further embodiment using a hydraulic actuator. Figure 15A shows the delivery device tip in its closed state. A nominal pressure PI is applied to a hydraulic actuator piston component 1501. The delivery device tip comprises a rear sheath 1502 having a stepped shape that is slidably retractable with regards to the piston. A tip sheath component 1503 is coupled to the piston.

In Figure 15B, the rear sheath is displaced proximally to deploy the stabilization arches (and optionally the upper crown). No hydraulic action is yet used.

In Figure 15C, an increased hydraulic pressure is applied to drive the piston distally. The piston may have a simple sliding action, or it may be guided by rotating in a screw thread. The tip sheath is displaced distally by the moving piston. The lower crown (and upper if not already uncovered) is uncovered by the tip sheath.

In Figure 15D, while maintaining the higher hydraulic pressure, the rear sheath is slid distally to close the gap between the sheaths. The delivery device then has a smooth surface allowing retraction through the valve. Alternatively, suction can be applied to draw back the distal sheath through the deployed valve.

In some embodiments (in which fluid action might or might not be used), the delivery system is configured to enable the distal sheath to be pulled back atraumatically through the valved-stent after deployment of the valved-stent, while the distal sheath remains in an open position or condition with respect to the delivery system. In the open condition, a space or gap may exist between, e.g. distal and proximal sheaths, and thus there may be a discontinuity or edge in the outer surface.

In some embodiments, a stent delivery system may employing an atraumatic balloon or cuff that can be deployed under fluid pressure to cover, or render less abrupt, the edge of the distal sheath. In an initial state, the cuff may be stowed in a compact form adjacent to the tip component. One edge of the cuff may be coupled to, or move with, the tip component and/or the distal sheath. Another edge of the cuff may be coupled to, or move with, the stent holder or a tube carrying the stent-holder. When the distal sheath is advanced distally as part of the deployment process, the cuff distends. An annular space between the stent-holder-carrying tube and an inner tube (e.g. coupled to the tip component) provides a fluid supply conduit to the cuff. Once the valved-stent has been deployed, inflation fluid may be supplied via the fluid supply conduit to inflate the cuff. The cuff may expand or distend to at least partly cover or shield the edge of the distal sheath, enhancing atraumicity when the distal sheath is to be pulled back in an open condition. For example, the cuff may define a doughnut-like, or bulged, shape shielding the edge of the distal sheath.

### Stents and Stented Valves

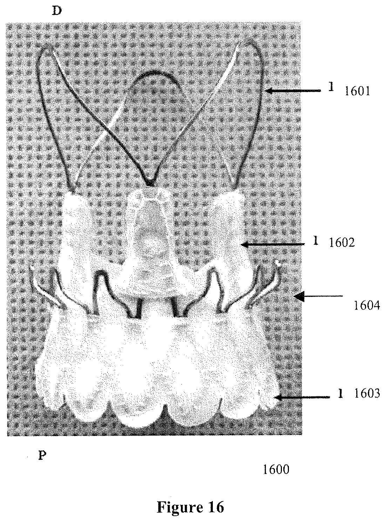

FIG. 16 illustrates an aortic bioprosthesis or stented replacement valve 1600 according to some embodiments. The stent component 1601 supports a replacement biological valve prosthesis 1602. In some embodiments, the valved-stent comprises the following elements: a valve 1602 (e.g., biologic porcine valve) which regulates the blood flow between the left ventricle and the aorta; a self expandable Nitinol stent 1601 acting as an anchoring structure within the native aortic annulus for the biological valve which is sutured on; and a double skirt 1603 (e.g., double polyester (PET) skirts) sutured on the inner and outer surface of the stent to reinforce the biological porcine valve and facilitate the leak-tightness of the implant.

The stent 1601 of the replacement valve may be self-expanding being formed from a suitable shape memory or superelastic material or combination of materials (e.g., nitinol). The stent is manufactured according to any know method in the art. In some embodiments, the stent is manufactured by laser cutting a tube or single sheet of material (e.g., nitinol). For example, the stent may be cut from a tube and then step-by-step expanded up to its final diameter by heat treatment on a mandrel. In some embodiments, the stent is manufactured by laser cutting from a tube of suitable shape memory or superelastic material or combination of materials (e.g., nitinol). Heat forming treatments may be applied, according to the current state-of-art , in order to fix the final shape of the stent. As another example, the stent may be cut from a single sheet of material, and then subsequently rolled and welded to the desired diameter.

Figure 2 illustrates the stent component of a aortic bioprosthesis or stented replacement valve 1600 according to some embodiments. The stent component 1601 defines a first (e.g., proximal) end and a second (e.g., distal) end and may be described as having one or more of 5 predominant features or sections that include: stabilization loops 1; commissural posts 2; upper (first) anchoring crown 3; lower (second) anchoring crown/portion 4; and inflow hooks 5.

Viewed alternatively, the stent component 1601 may be described as having one or more of: a distal stent section defining the distal end; a proximal anchoring section defining the proximal end; and an upper (first) crown section. The distal stent section may comprise the stabilization loop (section) 1 and commissural post (section) 2. The proximal anchoring section may comprise the lower (second) anchoring crown/portion 4. The upper (first) crown section may comprise the upper anchoring crown 3. The upper crown section may comprise a first divergent portion that diverges outwardly in a direction towards the distal end. The first crown section may have a free end. The free end may be proximal of the distal end of the stent and/or distal of the proximal end of the stent.

The stabilization loops 1 define the outflow section of the stent component (relative to main bloodflow direction in the native valve), and includes a generally divergent (e.g., conical) shape, with the conical curvature oriented in generally the same direction as the curvature of the upper anchoring crown 3. In some embodiments, the stabilization loops 1 includes a plurality of (e.g., 2, 3, 4, 5, 6, or more) larger arches generally in referred position to the arches in the commissural posts 2. In some embodiments, these larger arches are the first components of the stent to be deployed during the distal-to-proximal release of the aortic bioprosthesis or stented replacement valve 1600 from a first, unexpanded configuration to a second, expanded configuration.

In some embodiments, at least one of the deployed arches 1 engages the ascending aorta thereby orientating the delivery system/valved-stent longitudinally within the aorta/aortic annulus, thus preventing any tilting of the implanted valved-stent 1600. The stent 1601 may also include a radiopaque marker on or close to the distal end of one of the arches to aid in tracking the placement of the stent during implantation. The radial force of the stabilization loops 1 may be increased by adjusting the length and angle of the stabilization loops 1. In some embodiments, the tip of the elements forming the upper anchoring crown 3 and/or the stabilization loops 1 may be bent towards the longitudinal axis of the stent thereby avoiding potential injury of the sinus of vasalva (see e.g., FIG. 2). The free area between the stabilization loops 1 may be adjusted (i.e., increased or decreased) to improve the blood flow to the coronary arteries. This section of the stent may be attached to the anchoring crown section.

The commissural posts 2 is the portion of the stent to which the valve prosthesis 102 is attached. In some embodiment, commissural posts 2 includes a plurality (e.g., 2, 3, 4, 5, 6, or more) of arches (or other type of structure, e.g., post) for the fixation of the prosthetic valve commissures. In some embodiments, the commissural posts 2 may be designed with an asymmetrical shape (not shown) , in order to easily identify under fluoroscopy, the three-dimensional position of each prosthetic commissure. In some embodiments, the commissural posts 2 may be designed with dot markerbands to identify their respective position with regard to the ostium of the coronary arteries.

The upper anchoring crown 3 section may include a generally divergent portion. The divergent portion may have any suitable shape, such as conical, or flared with a nonuniform angle of divergence with respect to the central axis (e.g. domelike or trumpet-mouth) giving a convex or concave divergence, or a combination of any of these. The conical/divergent angle or curvature may be oriented in the opposite direction to the angle or curvature of lower anchoring crown 4 or proximal anchoring stent section 4. Due to its geometry, upper anchoring crown 3 creates a form fit with the supra-valvular apparatus and the native leaflets of the aortic valve. Therefore it prevents the migration of the valved-stent towards the left ventricle (migration of the implant during diastole). Furthermore, the upper anchoring crown 3 provides a radial force that creates an additional friction fit against the aortic annulus plus native leaflets. In some embodiments, the tips of crown elements 3 may be bent to form a cylindrical surface, thus reducing risks of sinus perforation.

Due to its geometry, the lower anchoring crown 4 section creates a form fitting with the inflow of an aortic valve (for example) and therefore prevents migration of the prosthesis towards the ascending aorta (migration of the implant towards the ascending aorta during systole). This section defines the proximal end P of the stent component (relative to a native valve, or heart or ventricle). The and is generally conically shaped. In some embodiments, the inflow edge maybe bended inward to avoid injuries at the level of the sub-valvular apparatus. Furthermore, the lower anchoring crown 4 provides a radial force that creates an additional friction fit against the inflow tract/aortic annulus.

Some embodiments may further include inflow-edge hooks 5, which assists the fixation of the aortic bioprosthesis to the delivery system (thru the stent holder) during the release procedure.

In some embodiments, the anchoring of the aortic bioprosthesis or stented replacement valve 1600 within the native calcified aortic annulus relies on two different aspects: form fitting based on the shape and features of the stent (e.g., by the joined shape of section 3 and section 4); and friction fitting based on the radial force applied by the self collapsible stent. The anatomical match between the stent and the aortic root is illustrated in Figure 3.

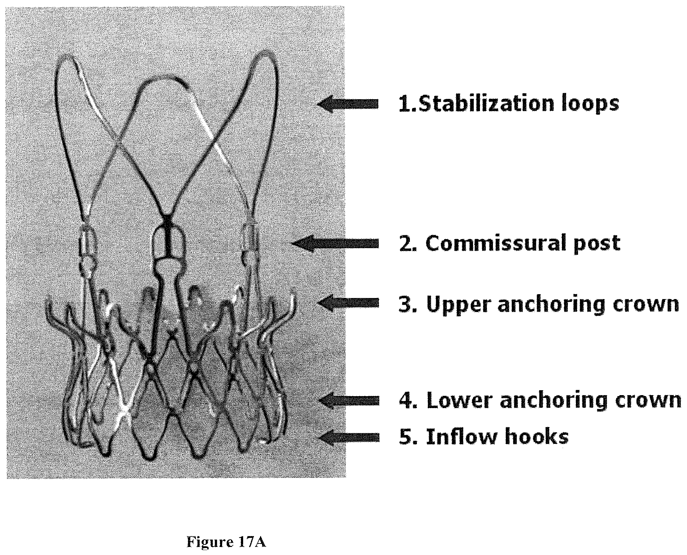

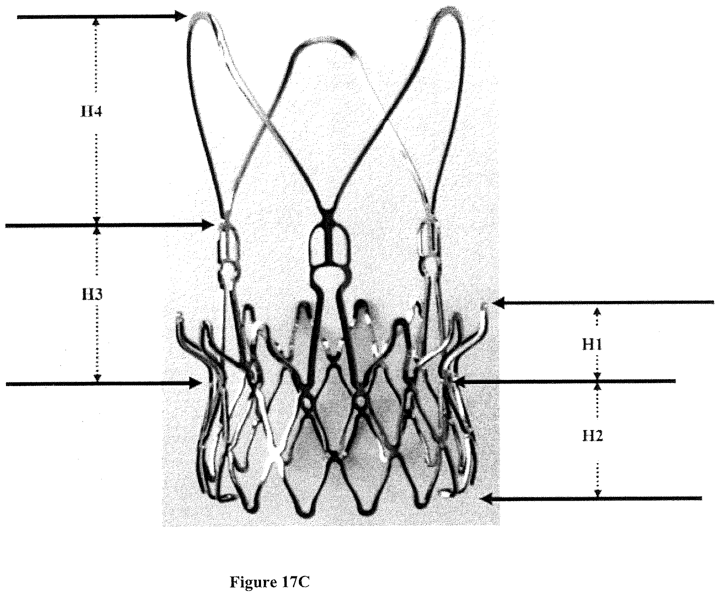

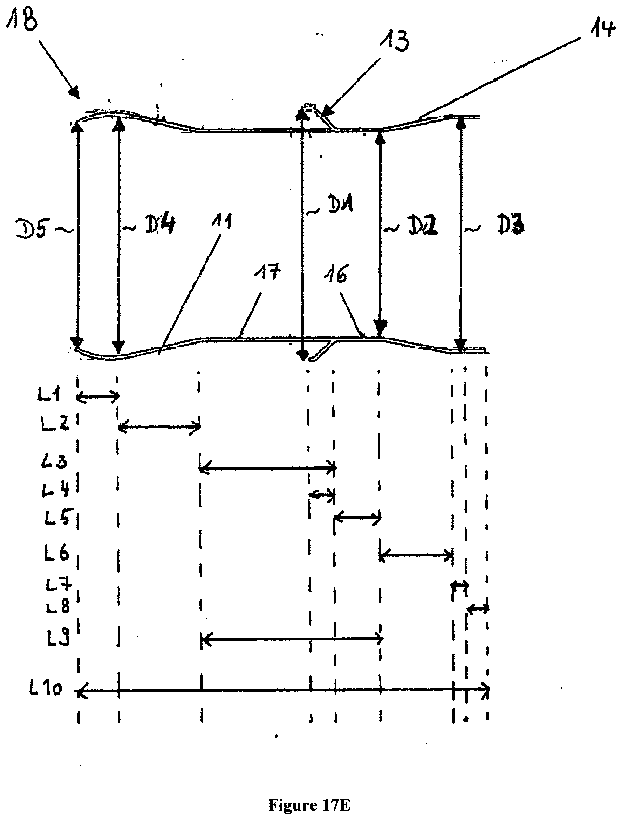

As shown in figure 17E in some embodiments there is a cylindrical section 16 between the upper conical crown 13 and the lower conical crown 14, The cylindrical section 16 may further extend to form commissural posts, such that the axial profile shows a further cylindrical section 17 between the upper conical crown 13 and the stabilisation loops 11.

The distal part 18 of the stabilisation loops 11 is inclined inwardly, such that the arms of the stabilisation loops 11 are bulged to allow the distal stent section adapting at the inside of the aorta.

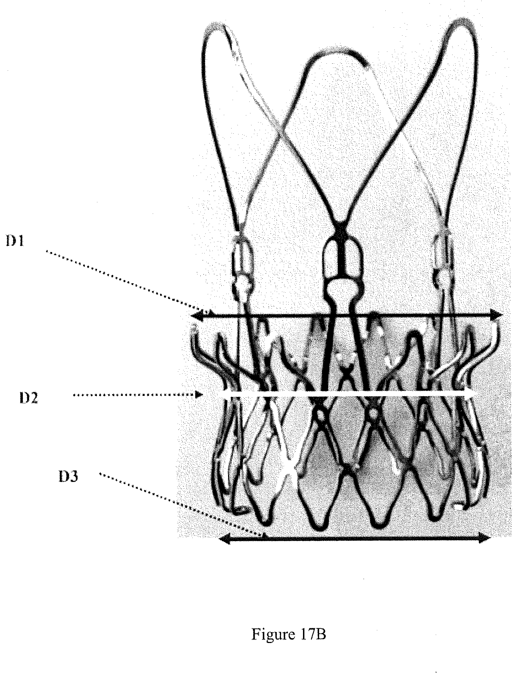

Figures 17B to 17D are provided to illustrate the dimensions of the stent component. D3 represents the diameter of the most proximal edge of the stent component in the expanded configuration. D2 represents the diameter of the stent component at the juncture between the upper and lower anchoring crowns. H2 represents the axial distance between the planes of the diameters D2 and D3 in the expanded configuration. DI represents the diameter of the most distal edge of the upper anchoring crown of the stent component in the expanded configuration. HI represents the axial distance between the planes of the diameters DI and D2 in the expanded configuration.

The length of H2 may be between about 3 to about 15 mm (e.g., about 3 mm, about 4 mm, about 5 mm, about 6 mm, about 7 mm, about 8 mm, about 9 mm, about 10 mm, about 11 mm, about 12 mm, about 13 mm, about 14 mm, and about 15 mm). The length of H2 may been adjusted depending on the intended application of the stent of valved-stent. For example, the length of H2 may range from about 3 to about 5 mm, about 3 to about 7 mm, about 3 to about 12 mm, about 3 to about 15 mm, about 3 to about 20 mm, about 5 to about 10 mm, about 5 to about 12 mm, about 5 to about 15 mm, about 7 to about 10 mm, about 7 to about 12 mm, about 7 to about 15 mm, about 10 to about 13 mm, about 10 to about 15 mm, or about 7 to about 20 mm. For example, the length of this section may be on the smaller end of the scale to avoid potential conflict with a cardiac valve, such as the mitral valve.

The diameter at D3 may be between about 22 mm to about 40 mm (e.g., about 22 mm, about 23 mm, about 24 mm, about 25 mm, about 26 mm, about 27 mm, about 28 mm, about 29 mm, about 30 mm, about 31 mm, about 32 mm, about 33 mm, about 34 mm, about 35 mm, about 36 mm, about 37 mm, about 38 mm, about 39 mm, and about 40 mm). This diameter D3 may been adjusted depending on the intended application of the stent of valved-stent. Thus, the diameter D3 in the expanded configuration may be from between about 15 mm to about 50 mm, from between about 15 mm to about 40 mm, from between about 20 mm to about 40 mm, from between about 24 mm to about 40 mm, from between about 26 mm to about 40 mm, from between about 28 mm to about 40 mm, from between about 30 mm to about 40 mm, from between about 32 mm to about 40 mm, from between about 34 mm to about 40 mm, from between about 36 mm to about 40 mm, from between about 38 mm to about 40 mm, from between about 22 mm to about 38 mm, from between about 22 mm to about 36 mm, from between about 22 mm to about 34 mm, from between about 22 mm to about 32 mm, from between about 22 mm to about 30 mm, from between about 22 mm to about 28 mm, from between about 24 mm to about 34 mm, from between about 25 mm to about 35 mm, or from between about 25 mm to about 30 mm.

The diameter of the stent component D2 may be between about 20 mm to about 30 mm (e.g., about 20 mm, about 21 mm, about 22 mm, about 23 mm, about 24 mm, about 25 mm, about 26 mm, about 27 mm, about 28 mm, about 29 mm, and about 30 mm). This diameter of the stent component D2 may been adjusted depending on the intended application of the stent of valved-stent. For example, this diameter of the stent component D2 may be sized according to the shape of the annulus of the cardiac valve. Thus, the diameter of the stent component D2 may be from between about 15 mm to about 40 mm, from between about 15 mm to about 30 mm, from between about 18 mm to about 35 mm, from between about 22 mm to about 30 mm, from between about 24 mm to about 30 mm, from between about 26 mm to about 30 mm, from between about 28 mm to about 30 mm, from between about 22 mm to about 28 mm, from between about 22 mm to about 26 mm, from between about 20 mm to about 24 mm, from between about 20 mm to about 26 mm, from between about 20 mm to about 28 mm, and from between about 22 mm to about 32 mm. The diameter DI may be between about 22 mm to about 40 mm (e.g., about 22 mm, about 23 mm, about 24 mm, about 25 mm, about 26 mm, about 27 mm, about 28 mm, about 29 mm, about 30 mm, about 31 mm, about 32 mm, about 33 mm, 34 mm, 35 mm, 36 mm, 37 mm, about 38 mm, about 39 mm, and about 40 mm). This diameter DI may been adjusted depending on the intended application of the stent of valved-stent. Thus, the diameter in the expanded configuration DI may be from between about 15 mm to about 50 mm, from between about 15 mm to about 40 mm, from between about 20 mm to about 40 mm, from between about 24 mm to about 40 mm, from between about 26 mm to about 40 mm, from between about 28 mm to about 40 mm, from between about 30 mm to about 40 mm, from between about 32 mm to about 40 mm, from between about 34 mm to about 40 mm, from between about 36 mm to about 40 mm, from between about 38 mm to about 40 mm, from between about 22 mm to about 38 mm, from between about 22 mm to about 36 mm, from between about 22 mm to about 34 mm, from between about 22 mm to about 32 mm, from between about 22 mm to about 30 mm, from between about 22 mm to about 28 mm, from between about 24 mm to about 34 mm, from between about 25 mm to about 35 mm, or from between about 25 mm to about 30 mm.

The length of HI is between about 3 to about 10 mm (e.g., about 3 mm, about 4 mm, about 5 mm, about 6 mm, about 7 mm, about 8 mm, about 9 mm, and about 10 mm). The length of HI may be adjusted depending on the intended application of the stent of valved-stent. For example, the length of H2 may range from about 3 to about 5 mm, about 3 to about 15 mm, about 3 to about 20 mm, about 5 to about 10 mm, about 7 to about 10 mm, about 7 to about 12 mm, about 7 to about 15 mm, about 10 to about 13 mm, about 5 to about 15 mm, about 7 to about 20 mm. For example, the length of this section may be on the smaller end of the scale to avoid potential conflict with the sinus of Valsalva.

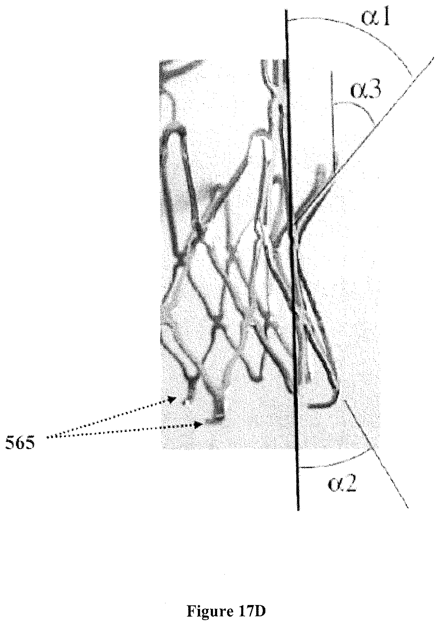

Figure 17D is provided to illustrate the angles of the anchoring crowns. The al angle defines the angle of the upper anchoring crown of the stent component in the expanded configuration. The a2 angle defines the angle of the lower anchoring crown of the stent component in the expanded configuration. The a3 angle defines the angle of bending of the tip, which is done so as to prevent injuries of sinus.

The al angle may be between from about 0 degree to about 90 degree (e.g., about 10 degree, about 15 degree, about 20 degree, about 25 degree, about 30 degree, about 35 degree, about 40 degree, about 45 degree, about 50 degree, about 55 degree, about 60 degree, about 65 degree, about 70 degree, about 75 degree, and about 80 degree). The al angle may be between from about 20 degree to about 70 degree, most preferable between from about 30 degree to about 60 degree. According to some embodiments, the oil angle is between from about 20 degree to about 80 degree, between from about 20 degree to about 60 degree, between from about 20 degree to about 50 degree, between from about 20 degree to about 45 degree, between from about 40 degree to about 60 degree, between from about 45 degree to about 60 degree, between from about 30 degree to about 50 degree, between from about 30 degree to about 45 degree, between from about 30 degree to about 40 degree, or between from about 25 degree to about 45 degree.

The 2 angle may be between from about 0 degree to about 50 degree (e.g., about 5 degree, about 10 degree, about 15 degree, about 20 degree, about 25 degree, about 30 degree, about 35 degree, about 40 degree, about 45 degree, and about 50 degree). The 2 angle may be between from about 10 degree to about 40 degree, most preferable between from about 10 degree to about 30 degree. According to some embodiments, the 2 angle is between from about 5 degree to about 45 degree, between from about 5 degree to about 40 degree, between from about 5 degree to about 30 degree, between from about 5 degree to about 25 degree, between from about 5 degree to about 20 degree, between from about 5 degree to about 15 degree, between from about 10 degree to about 20 degree, between from about 10 degree to about 25 degree, between from about 10 degree to about 30 degree, between from about 10 degree to about 40 degree, between from about 10 degree to about 45 degree, between from about 15 degree to about 40 degree, between from about 15 degree to about 30 degree, between from about 15 degree to about 25 degree, between from about 20 degree to about 45 degree, between from about 20 degree to about 40 degree, or between from about 20 degree to about 30 degree.

The 3 angle may be between from about 0 degree to about 180 degree (e.g., about 5 degree, about 10 degree, about 15 degree, about 20 degree, about 25 degree, about 30 degree, about 35 degree, about 40 degree, about 45 degree, about 50 degree, about 55 degree, about 60 degree, about 65 degree, about 70 degree, about 75 degree, about 80 degree, about 85 degree, about 90 degree, about 95 degree, about 100 degree, about 105 degree, about 110 degree, about 115 degree, about 120 degree, about 125 degree, about 130 degree, about 135 degree, about 140 degree, about 145 degree, about 150 degree, about 155 degree, about 160 degree, about 165 degree, about 170 degree, about 175 degree, and about 180 degree). According to some embodiments, the 3 angle is between from about 45 degree to about 90 degree, between from about 45 degree to about 180 degree, between from about 60 degree to about 90 degree, between from about 45 degree to about 120 degree, between from about 60 degree to about 120 degree, between from about 90 degree to about 120 degree, be- tween from about 90 degree to about 180 degree, or between from about 120 degree to about 180 degree.

The length of the upper anchoring crown 3 and commissural posts section 2 of the stent component H3 is between about 3 to about 50 mm (e.g., about 3 mm, about 4 mm, about 5 mm, about 6 mm, about 7 mm, about 8 mm, about 9 mm, about 10 mm, about 11 mm, about 12 mm, about 13 mm, about 14 mm, about 15 mm, about 20 mm, about 22 mm, about 24 mm, about 25 mm, about 26 mm, about 28 mm, about 30 mm, about 32 mm, about 34 mm, about 36 mm, about 38 mm, about 40 mm, about 42 mm, about 44 mm, about 45 mm, about 46 mm, about 48 mm, and about 50 mm). The length of H3 may been adjusted depending on the intended application of the stent of valved-stent. For example, the length of H3 may range from about 3 to about 40 mm, about 3 to about 30 mm, about 3 to about 20 mm, about 3 to about 10 mm, about 10 to about 50 mm, about 10 to about 40 mm, about 10 to about 30 mm, about 10 to about 20 mm, about 15 to about 50 mm, about 15 to about 40 mm, about 15 to about 30 mm, about 20 to about 50 mm, about 20 to about 40 mm, about 20 to about 30 mm, about 15 to about 50 mm, about 25 to about 50 mm, about 30 to about 50 mm, about 40 to about 50 mm, about 15 to about 40 mm, about 25 to about 40 mm, or about 30 to about 40 mm.

The length of the stabilization loops 1 of the stent component H4 is between about 5 to about 50 mm (e.g., about 5 mm, about 6 mm, about 7 mm, about 8 mm, about 9 mm, about 10 mm, about 11 mm, about 12 mm, about 13 mm, about 14 mm, about 15 mm, about 20 mm, about 22 mm, about 24 mm, about 25 mm, about 26 mm, about 28 mm, about 30 mm, about 32 mm, about 34 mm, about 36 mm, about 38 mm, about 40 mm, about 42 mm, about 44 mm, about 45 mm, about 46 mm, about 48 mm, and about 50 mm). The length of H4 may been adjusted depending on the intended application of the stent of valved-stent. For example, the length of H4 may range from about 5 to about 40 mm, about 5 to about 30 mm, about 5 to about 20 mm, about 5 to about 10 mm, about 10 to about 50 mm, about 10 to about 40 mm, about 10 to about 30 mm, about 10 to about 20 mm, about 15 to about 50 mm, about 15 to about 40 mm, about 15 to about 30 mm, about 20 to about 50 mm, about 20 to about 40 mm, about 20 to about 30 mm, about 15 to about 50 mm, about 25 to about 50 mm, about 30 to about 50 mm, about 40 to about 50 mm, about 15 to about 40 mm, about 25 to about 40 mm, or about 30 to about 40 mm.

The 4 and 5 angles represent the offset angle from a longitudinal axis of the stabilization loops 1 of the stent component in the expanded configuration. If the stabilization arches are directed away from the center of the stent, the 4 angle is used. If the stabilization arches are directed toward from the center of the stent, the 5 angle is used.

The 4 angle is preferably between from about 0 degree to about 60 degree (e.g., about 5 degree, about 10 degree, about 15 degree, about 20 degree, about 25 degree, about 30 degree, about 35 degree, about 40 degree, about 45 degree, about 50 degree, about 55 degree, and about 60 degree). According to some embodiments, the 4 angle is between from about 20 degree to about 60 degree, between from about 30 degree to about 60 degree, between from about 40 degree to about 60 degree, between from about 45 degree to about 60 degree, between from about 30 degree to about 50 degree, between from about 30 degree to about 45 degree, between from about 20 degree to about 40 degree, or between from about 15 degree to about 45 degree.

The 5 angle is preferably between from about 0 degree to about 20 degree (e.g., about 5 degree, about 10 degree, about 15 degree, and about 20 degree). According to some embodiments, the 5 angle is between from about 5 degree to about 20 degree, between from about 10 degree to about 20 degree, between from about 15 degree to about 20 degree, between from about 0 degree to about 15 degree, between from about 0 degree to about 10 degree, between from about 5 degree to about 15 degree, between from about 10 degree to about 15 degree, or between from about 10 degree to about 20 degree.

According to some embodiments, there is provided a replacement valve comprising a valve component and a stent component, wherein the stent component comprises a lower anchoring crown, an upper anchoring crown, a commissural post section, and stabilization loops. The conical body of the lower anchoring crown may slope outwardly from an inner diameter D2 to an outer diameter D3 in the direction of the proximal end, wherein the inner diameter D2 may be between about 20 mm to about 27 mm, and wherein the outer diameter D3 may be between about 26 mm to about 33 mm. The axial distance between the planes of the diameters D2 and D3 in the expanded configuration (H2) may be between about 7 to about 11 mm, wherein the outward slope of the lower anchoring crown is defined by an angle 2, which may be between from about 15 degree to about 25 degree. The conical body of the upper anchoring crown may slope outwardly from an inner diameter D2 to an outer diameter DI in the direction of the distal end, wherein the inner diameter D2 may be between about 20 mm to about 27 mm, and wherein the outer diameter DI may be between about 26 mm to about 33 mm. The axial distance between the planes of the diameters D2 and DI in the expanded configuration (HI) may be between about 4 to about 8 mm. The outward slope of the lower anchoring crown may be defined by an angle oil, which may be between from about 45 degree to about 65 degree. The end of the upper anchoring crown may form a tip, wherein the tip is bent inwardly toward the longitudinal axis at an angle 3. The angle 3 may be between from about 45 degree to about 65 degree. The length of the combined upper anchoring crown and commissural posts of the stent component (H3) may be between about 11 to about 15 mm. The length of the stabilization loops of the stent component (H4) may be between about 14 to about 30 mm (preferably up to about 22 mm) ; wherein the stabilization loops of the stent component expands outwardly at an angle 4 from a longitudinal axis toward the second distal end of the replacement valve. The angle 4 may be between about 5 degree to about 15 degree.

Some embodiments may comprise a replacement valve comprising a valve component and a stent component, wherein the stent component comprises a lower anchoring crown, an upper anchoring crown, a commissural post section, and stabilization loops. The conical body of the lower anchoring crown may slope outwardly from an inner diameter D2 to an outer diameter D3 in the direction of the proximal end. The inner diameter D2 may be between about 21 mm to about 26 mm, and the outer diameter D3 may be between about 27 mm to about 33 mm. The axial distance between the planes of the diameters D2 and D3 in the expanded configuration (H2) may be between about 8 to about 12 mm. The outward slope of the lower anchoring crown may be defined by an angle 2, which may be between from about 15 degree to about 25 degree. The conical body of the upper anchoring crown may slope outwardly from an inner diameter D2 to an outer diameter DI in the direction of the distal end. The inner diameter D2 may be between about 21 mm to about 26 mm, and the outer diameter DI may be between about 27 mm to about 32 mm. The axial distance between the planes of the diameters D2 and DI in the expanded configuration (HI) may be between about 4 to about 8 mm. The outward slope of the lower anchoring crown is defined by an angle I, which may be between from about 45 degree to about 65 degree. In some embodiments, the end of the upper anchoring crown forms a tip, wherein the tip is bent inwardly toward the longitudinal axis at an angle 3, which may be between from about 45 degree to about 65 degree. The length of the combined upper anchoring crown and commissural posts section of the stent component (H3) may be between about 13 to about 17 mm. The length of the stabilization loops and of the stent component (H4) may be between about 15 to about 23 mm. In some embodiments, the stabilization loops of the stent component expands outwardly at an angle 4 from a longitudinal axis toward the second distal end of the replacement valve. The angle 4 is between about 5 degree to about 15 degree. According to some embodiments, there is provided a replacement valve comprising a valve component and a stent component, wherein the stent component comprises a lower anchoring crown, an upper anchoring crown, a commissural post section, and stabilization loops. The conical body of the lower anchoring crown may slope outwardly from an inner diameter D2 to an outer diameter D3 in the direction of the proximal end. The inner diameter D2 may be between about 22 mm to about 27 mm, the outer diameter D3 may be between about 28 mm to about 34 mm, and the axial distance between the planes of the diameters D2 and D3 in the expanded configuration (H2) may be between about 9 to about 13 mm. The outward slope of the lower anchoring crown may be defined by an angle 2, and wherein 2 is between from about 15 degree to about 25 degree. The conical body of the upper anchoring crown slopes outwardly from an inner diameter D2 to an outer diameter DI in the direction of the distal end, wherein the inner diameter D2 may be between about 22 mm to about 27 mm, and wherein the outer diameter DI may be between about 28 mm to about 33 mm. The axial distance between the planes of the diameters D2 and DI in the expanded configuration (HI) may be between about 4 to a- bout 8 mm; wherein the outward slope of the lower anchoring crown is defined by an angle I, which may be between from about 45 degree to about 65 degree. The end of the upper anchoring crown may form a tip, wherein the tip is bent inwardly toward the longitudinal axis at an angle 3, which may be between from about 45 degree to about 65 degree. The length of the combined upper anchoring crown and commissural post section of the stent component (H3) may be between about 15 to about 19 mm. The length of the stabilization loops and of the stent component (H4) may be between about 16 to about 24 mm. The stabilization loops of the stent component expand outwardly at an angle 4 from a longitudinal axis toward the second distal end of the replacement valve, wherein 4 is between about 5 degree to about 15 degree.

In some embodiments, multiple fixation elements (e.g., 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, etc. or 2 to 5, 2 to 10, 2 to 20, 2 to 30, 2 to 40, etc.) may be provided for holding the stent onto a catheter whereas a matching/complimentary element (e.g., stent holder with pins) may be attached to the delivery device. The design of the multiple fixation elements (e.g., forming "holes") may allow for the fixation of the stent onto the catheter only when the stent is crimped. The fixation may release automatically when the stent starts to expand. That is, the shape of the stent in the unexpanded state is designed to have holes or free areas that can be used to couple the stent with a stent holder. When the stent is expanded, the expanded configuration is absent suchs holes or free spaces and thus the stent automatically becomes uncoupled or releases from the stent holder upon expansion.

The stent component may further include at least one or a plurality (e.g., 2, 3, 4, 5, 6, 7, 8, 9, 10, etc.) of attachment elements at the proximal end of the stent, wherein the attachments elements are capable of mating with the stent holder 560 of the delivery device 500. The attachment elements may include a crochet-like configuration that engages, for example, a groove or other opening within the stent holder 560. Such attachment elements may be formed in the shape of a bent, or curved angled member (e.g., an "L" or "J" like shape). See FIG 2D. In some embodiments, such attachment elements may be a hook (e.g., a "J" like shape). In the embodiment illustrated in Fig. 2D, the attachment element may be provided in an angled shape, for example, that extends from the body of the stent inwardly toward a central, longitudinal axis of the stent. The opening in the stent holder 560 (e.g., groove) may allow for a safe release of the stent upon rotation of the delivery system (e.g., a portion, all or members thereof - e.g., rotation of the stent holder). For example, when rotating the delivery system/stent holder, the end of the attachment element slides onto the surface "S" (e.g. a ramp extending in the circumferential direction) and is thereby forced, according to some embodiments, to disengage the stent holder when reaching the edge "E" (e.g. radially outermost end of ramp).

Using the dimensions as referenced in Figure 17E, the stent components of the valved-stents according to some embodiments of the present disclosure may be classified into different categories of sizes, such as small, medium, and large. Thus, according to some embodiments, the stent components (or valved- stents) may be sized as small, medium, and large according to the following table.

| | **Small** | **Medium** | **Large** |
|---|---|---|---|
| D1 [mm] | 26.5- 28.5 | 28.8-30.8 | 30.9-32.9 |
| D2 [mm] | 21.6-23.6 | 23.6-26.6 | 25.6-27.6 |
| D3 [mm] | 24.8-26.8 | 27-29 | 29.1-31.1 |
| D4 [mm] | 26-28 | 28-30 | 30-32 |
| D5 [mm] | 24.1-26.1 | 26.4-28.4 | 28.5-30.5 |
| L1 [mm] | 3.5-4.9 | 3.5-4.9 | 3.5-4.9 |
| L2 [mm] | 10.0-11.2 | 9.9-11.1 | 9.7-10.9 |
| L3 [mm] | 13.9-14.9 | 15.1-16.1 | 16.1-17.1 |
| L4 [mm] | 3.0-4.0 | 3.0-4.0 | 3.0-4.0 |
| L5 [mm] | 4.5-5.5 | 4.5-5.5 | 4.5-5.5 |
| L6 [mm] | 7.1-8.1 | 7.5-8.5 | 7.7-8.8 |
| L7 [mm] | 1.0-2.0 | 1.0-2.0 | 1.0-2.0 |
| L8 [mm] | 1.9-2.9 | 1.9-2.9 | 1.9-2.9 |
| L9 [mm] | L3+L4 | L3+L4 | L3+L4 |

D1 represents the diameter of the stent component at the distal edge of the outwardly sloping upper conical crown in the expanded configuration.

D3 represents the diameter of the most proximal edge of the stent component in the expanded configuration.

D2 represents the diameter of the stent component at the cylindrical section between the upper and lower anchoring crowns in the expanded configuration.

D4 represents the diameter of the stent component at the junction between the outwardly and inwardly bended sections of the stabilization loops in the expanded configuration.

D5 represents the diameter of the stent component at the most distal edge of the stent in the expanded configuration.

L1 represents the axial length of the inwardly bended part of the stabilization loops. L2 represents the axial length of the outwardly sloping part of the stabilization loops.

L3 represents the axial length of the cylindrical section be- tween the upper conical crown and the stabilization arches.

L4 represents the axial length of the upper conical crown in the expanded configuration.

L5 represents the axial length of the cylindrical section between the upper conical crown and the lower conical crown.

L6 represents the axial length of the outwardly sloping part of the lower conical crown in the expanded configuration.

L7 represents the axial length of the cylindrical part of the lower conical crown.

L8 represents the axial length of the axial extensions forming attachment elements.

L9 represents the axial length of the cylindrical section between the stabilization arches and the lower conical crown. The total lengths L10 of the stent thus results in a range of 41 mm to 49 mm.

It has been observed in vivo that some embodiments of the stent component may allow for self-positioning of the replacement valve under diastolic pressure. Once delivered slightly above the aortic annulus, the valved-stent migrates toward the left ventricle due to the forces caused by the diastolic pressure until it reaches a stable position given by the shape / radial force of the anchoring crowns, the compliance of the aortic annulus, and presence of any calcification.

In some other embodiments, the presence of calcification deposits at the native valve may limit or prevent sliding movement of the valve from a release position to a different stable position. In that case, the stable position may be the same as the release position.

In some embodiments, a valved-stent suitable for implanting at a calcified native valve site comprises an upper (first) crown section comprising at least a portion diverging outwardly in a direction towards an aortic end of the valved-stent. The upper crown section may have a free end (e.g. proximal of the distal end of the stent component). The upper crown/divergent portion may have an angle of divergence (or inclination or conical angle) with respect to the stent axis of less than 60 degrees (preferably 50 degrees or less, preferably 45 degrees or less, for example 43-45 degrees) and/or have an axial length of less than 10mm (preferably less than 8mm, preferably less than 6mm, preferably less than 5 mm, for example, 3 - 4 mm). Such dimensions may be regarded as less sculpted than in some prior designs. The dimensions can nevertheless provide a reliable abutment surface to resist migration of the valved-stent towards the ventricle during ventricular diastole, without the upper crown being so large and/or having an aggressive angle of inclination that the positioning is likely to be affected adversely by the calcified deposits.

Additionally or alternatively, a valved-stent suitable for implanting at a calcified valve site comprises an upper (first) crown comprising at least a portion diverging outwardly in a direction towards and aortic end of the valved-stent. A substantially non-diverging region communicating with the narrow end of the diverging portion may extend therefrom in a direction towards the ventricular end of the valved-stent. The term "substantially non-diverging" may mean a divergence of no more than 10 degrees, preferably less than 8 degrees, preferably less than 6 degrees, preferably less than 5 degrees, preferably less than 4 degrees, and preferably zero degrees. The substantially non- diverging region may have an axial length of at least 1mm, preferably at least 2mm, preferably at least 3mm, preferably at least 4mm, for example, 4.5-5.5mm. Provision of such a substantially non-diverging region may enable the valved-stent to better accommodate calcified deposits where the valved-stent passes through the native valve and/or native annulus. The substantially non-diverging region may separate (at least a portion of) the upper (first) crown from (at least a portion of) the lower (second) crown. The substantially non-diverging section may form a part of the upper crown section and/or lower crown section.

Additionally or alternatively, a valved-stent suitable for implanting at a calcified valve site comprises a lower crown section. The lower crown section may comprise at least a portion diverging outwardly in a direction towards the ventricular end of the valved-stent. The lower crown and/or divergent portion may be provided at a portion of the valved-stent intended to be received at the ventricle, for engaging native tissue to resist migration of the valved-stent in a direction out of the ventricle. The divergent portion of the lower crown section may have an angle of divergence with respect to the valved-stent axis of between 10 degrees and 20 degrees (preferably 10-16 degrees, more preferably 10-15 degrees, more preferably 10-14 degrees, more preferably 10-13 degrees). Such an angle of divergence may be regarded as less sculpted than some prior designs. However, the angle permits the lower crown to function to resist migration, while being versatile in accommodating a wide range of calcifications without affecting function.

In one proposal, an upper crown of a valved-stent is provided that is not too big, the axial length L4 being between 3 and 4 mm and the angle oil of the upper crown being between 43° and 45°, as well as the length of the cylindrical part 16 being not too small, the length L5 being between 4.5-5.5 mm. Stents of this type do not block the coronary arteries or contact the sinus of the Vasalva, they reduce the risk of coronary occlusion and they even fit to a calcified annulus.

In a preferred embodiment the lower crown comprises a relatively small conical angle of about 10° to about 13° and a cylindrical proximal section with an axial length of about 1-2 mm. Stents of this type allow a homogeneous seating towards a calcified annulus and less turbulences within the valve inflow.

In a further preferred embodiment the stent stabilization loop are bent inwardly with a relatively big radius of the curvature to avoid injuries of the ascending aorta.

### Segmented Structures

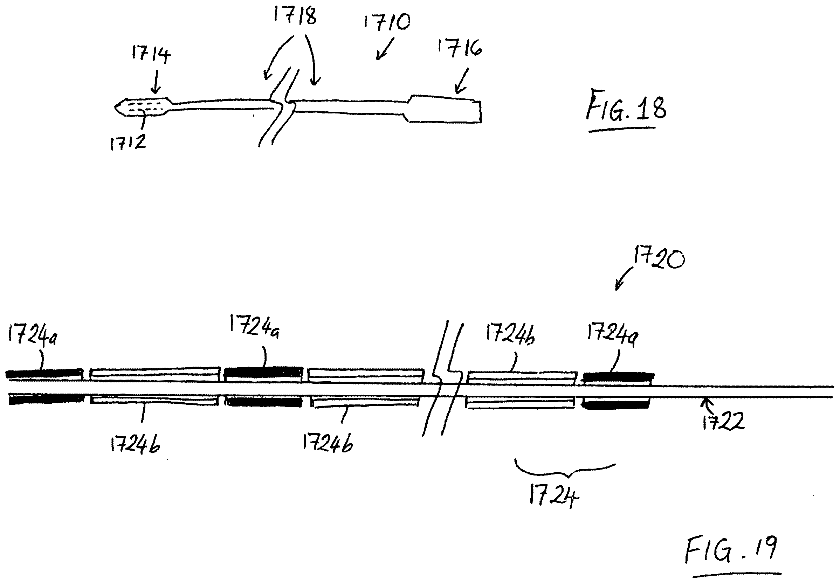

Referring to Fig. 18, a delivery catheter 1710 is illustrated for introduction into a patient's vasculature for delivering a cardiac stent-valve 1712 for implantation to replace an existing cardiac valve. The stent-valve 1712 may be delivered in a collapsed condition, and be expandable to a deployed condition at implantation. The stent-valve 1712 may be self-expanding, or it may be expandable by application of an expansion force. The stent-valve 1712 may, for example, be as described in WO 2009/053497 and/or as described above. However, many different types of stent-valve are known in the art, and the invention is not limited to a specific design of stent-valve 1712.

The delivery catheter generally comprises a distal portion 1714 for introduction into the vasculature, a proximal portion 1716 that may remain outside body, and a stem or barrel portion 1718 extending between the distal and proximal portions. The distal portion 1714 comprises an arrangement for carrying the stent- valve 1712 in a collapsed condition for delivery to the implantation site and operable to release or deploy the stent-valve 1712 in response to remote manipulation. The proximal portion 1716 comprises one or more manually operable controls for manipulating the distal portion 1714. The stem portion 1718 is configured to be:
(i) flexible to permit tracking of the delivery catheter along the patient's tortuous vasculature (including the extreme bend of the aortic arch) , and
(ii) capable of providing mechanical support for:
   (a) enabling the distal portion 1714 to be pushed and advanced along the vasculature by pushing force applied to the delivery catheter from outside the body; and
   (b) transmitting a differential, longitudinal displacement force from the proximal portion 1716 to the distal portion 1714 for manipulating the distal portion 1714.

In the prior art, the requirements (i) and (ii) above generally conflict with each other. It has been problematic to find materials for the delivery catheter, especially the stem portion 1718, without compromising at least one of the requirements (i) and (ii). For example, if the material is advantageously flexible to satisfy requirement (i), the material may lack sufficient rigidity for applying longitudinal forces within the stem. This can lead to the material deforming elastically, kinking or buckling, especially where elongate compression and/or elongate pushing force is applied though the material. Alternatively, if the material is relatively stiff to satisfy requirement (ii), this can compromise the ability of the delivery catheter to flex in order to follow a tortuous vasculature, or it may result in undesirable abrasive rubbing against the vassal wall (for example, especially where the catheter passes around the aortic arch, which is often highly stenosed and there is a risk of embolism resulting from calcification rubbed free of the vassal wall and released into the blood stream).

Referring to Fig. 18, some embodiments of the present invention address this issue by providing a structure and/or sub-assembly 1720 comprising a flexible support tube 1722 and a segmented tube 1724 nested one within the other. In the illustrated form, the support tube 1722 is arranged as a core, and the segmented tube 1724 as a sleeve around the core. However, the relative positions may be swapped if desired. The segments may include plural first segments 1724a having a first elastic modulus, and interposed at least one between plural second segmentsl724b having a second elastic modulus different from the first elastic modulus. In the illustrated form, the segments 1724 are arranged in a repeating sequence of a first segment 1724a inter- posed contiguously between second segments 1724b adjacent to the first segment 1724a. For example, the segments 1724 may define a continuous and/or repeating pattern of alternating (e.g. longitudinally alternating) first and second segments 1724a and 1724b, respectively. If desired, additional segments, e.g., third segments (not shown) of a third elastic modulus, may further be included as part of the sequence of segments 1724. By way of example, the first elastic modulus may be higher than the second elastic modulus, e.g., by a factor selected from: at least 10 times; at least 100 times; at least 500 times; at least 1000 times.

In Fig. 19, the segments 1724 are shown slightly spaced apart from each other, and from the surface of the support tube 1722, for ease of visualization. However, it may be appreciated that the segments 1724 may directly abut each other and/or may be dimensioned to form a close fit around the support tube 1722. The combination of the segments 1724 and support tube 1722 may define a generally integral structure without substantial play between individual segments and/or between the segments 1724 and the support tube 1722.

The provision of such segments 1724a and 1724b provides a structure that can meet better both of the above requirements (i) and (ii). The first segments 1724a (of relatively higher elastic modulus) provide stiffness enabling the structure 1720 to bear compression loads enabling a longitudinal and/or axial pushing force to be transmitted through the structure, without significant elastic deformation (e.g. compression) even when the structure follows a non-straight path. The first segments provide good column strength. The second segments 1724b (of relatively lower elastic modulus) provide flexibility between the first segments 1724a, thereby enabling the structure 1720 to retain good flexibility.

In some embodiments, the first segments 1724a are of metal, for example, stainless steel. In some embodiments, the second segments 1724b are of flexible plastics. The second segments 1724b are optionally of the same material as the support tube 1722.

The properties of the structure 1720 may also be adjusted by the length (s) of the first and second segments 1724a and 1724b. The segments 1724 may be generally elongate in the axial direction of the structure 1720. The first and/or second segments 1724a and 1724b may have a length of less than about 3 cm. The segments 1724a and 1724b may have the same length or different lengths. In the illustrated form, the first segments 1724a (of relatively higher elastic modulus) are shorter than the second segments 1724b. For example, the first segments may have a length of about 1cm and/or the second segments 1724b may have a length of about 2cm. The length (s) of the first and second segments 1724a and 1724b may be constant over the length of the structure 1720, or the length (s) may vary over the length of the structure 1720 to provide different properties over the length.

The segments 1724a and 1724b may optionally all be affixed to the support tube 1722 to define an integrated structure. Alternatively, respective segments 1724a and 1724b may be affixed in certain affixed zones of the structure 1720 leaving other segments captive between the affixed zones. For example, the affixed zones may be or include regions at the opposite ends of the structure 1720.

The structure 1720 may be assembled easily and cost effectively by sliding the segments 1724a and 1724b onto the support tube 1722, so that the structure does not add significantly to the cost of the delivery device.

The structure 1720 may define a tubular lumen 1726 therewithin, for passage therethrough of one or more other members. In some embodiments, the tubular lumen 1726 is a guide-wire receiving lumen for passage of a guide wire through the delivery system 1710.

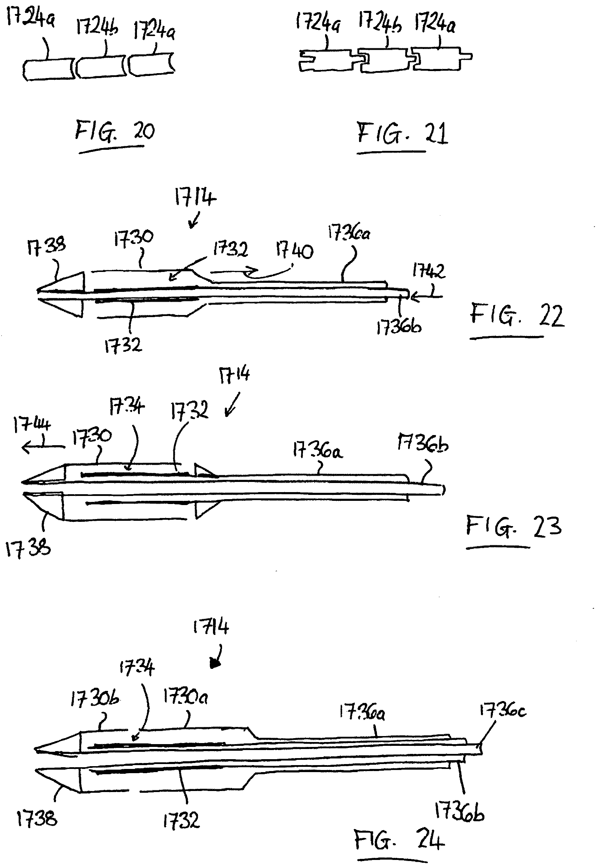

In the form illustrated in Fig. 19, the confronting ends of adjacent segments 1724 may be generally flat, such that ends abut each other face to face without any mechanical keying. Referring to Figs. 20 and 21, in some embodiments, the ends may include mechanical keying. For example, in Fig. 20, the end of one segment 1724 may fit partly within a recess or pocket of an adjacent segment 1724. The end of one segment may be beveled or rounded. Such an arrangement may optionally provide support for transmitting longitudinal compression force, while also facilitating bending articulation. Referring to Fig. 21, the mechanical keying may additionally or alternatively include non-rotation keying for transmitting torque from one segment between adjacent segments 1724.

Figs. 22-24 illustrate examples of different distal portion 1714 of the delivery device 1710, to show how the structure 1720 may advantageously be used. The distal portion 1714 may optionally comprise at least one containment sheath part 1730 and/or a stent-holder (indicated schematically at 1732). The distal portion may define a stent containing or stent attachment region 1734. The sheath part 1730 may be configured for containing a stent-valve 1712 (from Fig. 18) in a collapsed condition. For example, the sheath part(s) 1730 may encompass at least a portion of the stent-valve 1712 to contain the stent-valve and/or constrain the stent-valve 1712 against expansion. The stent- holder 1732 may comprise one or more elements, stops and/or surfaces configured to restrain the stent-valve 1712 against axial movement in at least one (and preferably both) axial direction (s). The distal portion 1714 may be configured to release the stent-valve 1712 from the stent containing region 1734 by displacement of the sheath part(s) 1730 relative to the stent- holder 1732. The displacement is driven by movement of respective control members at the proximal portion 1716, which movement is transmitted through the stem 1718 to the distal portion 1714. The stem portion 1718 comprises plural tubular members 1736 nested one within another, and relatively displaceable to transmit displacement forces from the proximal portion 1716 to the distal portion 1714. One or more of the tubular members 1736 may comprise the structure 1720 described above, which is especially useful as a tubular member for transmitting a longitudinal pushing, or compressive force to the distal portion 1714.

In the example of Fig. 22, the distal portion comprises a single sheath part 1730 that is displaceable in a proximal direction (indicated by arrow 1740) relative to the stent holder 1732 to open the stent containing region 1734. A distal tip 1738 may be fixed relative to the stent holder 1732. The tubular members 1736 include an (e.g. outer) tubular member 1736a coupled to the sheath part 1730. The structure 1720 may be used as an (e.g. inner) tubular member 1736b for restraining the stent holder 1732 by application of a longitudinal pushing force (indicated by arrow 1742) when the sheath part 1730 is pulled back by the outer tubular member 1736a.

In the example of Fig. 23, the distal portion comprises a single sheath part 1730 that is displaceable in a distal direction (indicated by arrow 1744) relative to the stent holder 1732 to open the stent containing region 1734. The distal tip 1738 may be fixed relative to the sheath part 1730. The tubular members may comprise an (e.g. outer) tubular member 1736a for restraining the stent holder 1732. The structure 1720 may be used as an (e.g. inner) tubular member 1736b coupled to the sheath part 1730 to apply the pushing force to the sheath part 1730.

In the example of Fig. 24, the distal portion comprises first and second sheath parts 1730a and 1730b that move in opposite directions to open the stent containing region 1734. The first sheath part 1730a is similar to the sheath illustrated in Fig 22. The second sheath part 1730b is similar to the sheath illustrated in Fig. 23. The tubular members 1736 include an (e.g. outer) tubular member 1736a coupled to the first sheath part 1732a, an (e.g. inner) tubular member 1736b coupled to the second sheath part 1732b, and an (e.g. central tubular member 1736c coupled to the stent holder 1732. The inner tubular member 1736b may optionally comprise a respective structure 1720 described above useful for transmitting a pushing force for advancing the second sheath part 1730b when opening the second sheath part 1730b. Additionally or alternatively, the central tubular member 1736c may optionally comprise a respective structure 1720 useful for transmitting a restraining (pushing) force to the stent holder 1732 when opening the first sheath part 1730a. The first and second sheath parts 1730a and 1730b may be configured for displacement individually in separate sequence, for example, for sequential release of the stent-valve.

Although the above description illustrates a delivery catheter for delivery a stent-valve, it will be appreciated that the same principles may be used in a flexible delivery catheter for delivering other types of stents. In particular, the delivery catheter may be suitable for stent grafts, for example, for treating a thoracic aortic aneurism and/or an abdominal aortic aneurism.

### Medical Uses

There are four valves in the heart that serve to direct the flow of blood through the two sides of the heart in a forward direction. On the left (systemic) side of the heart are: 1) the mitral valve, located between the left atrium and the left ventricle, and 2) the aortic valve, located between the left ventricle and the aorta. These two valves direct oxygenated blood coming from the lungs through the left side of the heart into the aorta for distribution to the body. On the right (pulmonary) side of the heart are: 1) the tricuspid valve, located between the right atrium and the right ventricle, and 2) the pulmonary valve, located between the right ventricle and the pulmonary artery. These two valves direct de- oxygenated blood coming from the body through the right side of the heart into the pulmonary artery for distribution to the lungs, where it again becomes re-oxygenated to begin the circuit anew.

Problems that can develop with heart valves consist of stenosis, in which a valve does not open properly, and/or insufficiency, also called regurgitation, in which a valve does not close properly. In addition to stenosis and insufficiency of heart valves, heart valves may need to be surgically repaired or replaced due to certain types of bacterial or fungal infections in which the valve may continue to function normally, but nevertheless harbors an overgrowth of bacteria on the leaflets of the valve that may embolize and lodge downstream in a vital artery. In such cases, surgical replacement of either the mitral or aortic valve (left-sided heart valves) may be necessary. Likewise, bacterial or fungal growth on the tricuspid valve may embolize to the lungs resulting in a lung abscess. In such cases replacement of the tricuspid valve even though no tricuspid valve stenosis or insufficiency is present.

Some embodiments of the presently claimed invention may be useful in a method for replacing a worn or diseased valve comprising transapically implanting a replacement valve, wherein the replacement valve is a valved-stent of the present disclosure. Accordingly, the replacement valve comprises a valve component and a stent component, wherein the valve component is connect to the stent component. Upon implantation, the replacement valve is positioned so that the annular groove receives the annulus of the worn or diseased cardiac valve.

In some cases, the valved-stents of the present disclosure may be designed to be self-positioning under diastolic pressure (i.e., permissible in vivo migration). The placement of the valved-stent may be upstream of the annulus, whereupon when the valved-stent will be locked into position once the annular groove of the stent component receives the annulus. Thus, some embodiments of the presently claimed invention may be useful in methods for implanting a replacement valve into a heart of a mammal comprising delivering a replacement valve to an implantation site of the heart of the mammal. The implantation site may comprise a release location and a final location; and the release location is spaced apart from the final location (and according to some embodiments, the spacing comprises a predetermined distance) in a blood upflow direction. Releasing the replacement valve at the release location, the replacement valve is able to slide into the final location, generally upon at least one beat of the heart subsequent to the replacement valve being released at the release location.

Some embodiments of the presently claimed invention may be useful in methods that when the replacement valve sliding into the final location, the replacement valve is substantially positioned to the final location.

Some embodiments of the presently claimed invention may be useful in a method for replacing an aortic valve within a human body. A valved-stent may be covered with a sheath in order to maintain the valved-stent in a collapsed configuration. The valved-stent may then may be inserted in the collapsed configuration into the human body without contacting the ascending aorta or aortic arch. The valved-stent may be partially expanded by sliding the sheath towards the left ventricle of the heart. This sliding of the sheath towards the left ventricle may cause expansion of a distal end of the valved-stent while the proximal end of the valved-stent remains constrained by the sheath. The sheath may be further slid towards the left ventricle of the heart in order to cause full expansion of the valved-stent. In some embodiments, the valved-stent may be recaptured prior to its full expansion by sliding the sheath in the opposite direction.

Some embodiments of the presently claimed invention may be useful in a method for cardiac valve replacement that includes releasing a distal end of a valved-stent from a sheath, where the distal end includes a radiopaque marker positioned thereon. The valved-stent is rotated, if necessary, to orient the valved-stent appropriately with respect to the coronary arteries (e.g., to prevent the commissures from facing the coronary arteries). Stabilization loops 1 of the valved- stent are released from the sheath, in order to cause the stabilization loops 1 to contact the aorta. A upper anchoring crown 3 of the valved-stent is released from the sheath, in order to cause the upper anchoring crown 3 to contact the native valve leaflets. A lower anchoring crown 4 of the valved-stent is released from the sheath, in order to cause the lower anchoring crown 4 to contact an annulus/inflow tract. The lower anchoring crown 4 may be the proximal section of the valved-stent such that releasing the lower anchoring crown 4 causes the valved- stent to be fully released from the sheath.

According to some embodiments, a replacement valve for use within a human body is provided, where the replacement valve includes a valve component and a stent component. The stent component also may be used without a connected valve as a stent. The stent devices of the present disclosure may use used to mechanically widen a narrowed or totally obstructed blood vessel, typically as a result of atherosclerosis. Accordingly, the stent devices of the present disclosure may use used is angioplasty procedures. These include: percutaneous coronary intervention (PCI), commonly known as coronary angioplasty, to treat the stenotic (narrowed) coronary arteries of the heart found in coronary heart disease; peripheral angioplasty, performed to mechanically widen the opening in blood vessels other than the coronary arteries.

Thus, it is seen that valved-stents (e.g., single-valved-stents and double-valved-stents) and associated methods and systems for surgery are provided. Although particular embodiments have been disclosed herein in detail, this has been done by way of example for purposes of illustration only, and is not intended to be limiting with respect to the scope of the appended claims, which follow.

## Claims

1. Cardiac valved stent delivery system for delivering a cardiac valved stent to the heart, comprising a cardiac valved stent and a cardiac valved stent delivery device,
wherein the delivery device comprises a tip and the tip comprises a tip sheath (1503) and a hydraulic actuator piston component (1501) which is coupled to the tip sheath (1503),
wherein the delivery device is configured to use hydraulic action which is transmitted to the hydraulic actuator piston component (1501) of the tip of the delivery device via a fluid-tight conduit to apply driving force to the hydraulic actuator piston component (1501) which distally displaces the tip sheath (1503),
wherein the tip comprises a rear sheath (1502) having a stepped shape that is slidably retractable with regards to the piston.

2. The delivery system of claim 1, wherein the delivery device is configured to distally slide the piston or rotate the piston in a screw thread causing its distal displacement.

3. The delivery system of any of claims 1 or 2, wherein the valved stent is self-expandable.

4. The delivery system of any one of claims 1 to 3, wherein the delivery device is configured to allow a minimally-invasive surgical approach whereby valve replacement surgery is performed on a beating heart without the need for an open-chest cavity and heart-lung bypass.

5. The delivery system of any one of claims 1 to 4, wherein the cardiac valved stent is a valved stent for replacing an aortic valve.

6. The delivery system of any one of claims 1 to 5, wherein the cardiac valved stent is a valved stent for replacing a mitral valve.

7. The delivery system of any one of claims 1 to 6, wherein the delivery system is configured for transapical or for transvascular delivery of the valved stent.

## Patentansprüche

1. Kardialklappenstent-Zuführsystem zum Zuführen eines Kardialklappenstents zu einem Herz, umfassend einen Kardialklappenstent und eine Kardialklappenstent-Zuführvorrichtung,
wobei die Zuführvorrichtung eine Spitze umfasst und die Spitze eine Spitzenhülse (1503) und eine Hydraulikbetätigungskolbenkomponente (1501) umfasst, die an die Spitzenhülse (1503) gekoppelt ist,
wobei die Zuführvorrichtung dazu ausgestaltet ist, Hydraulikwirkung zu nutzen, die über eine fluiddichte Leitung an die Hydraulikbetätigungskolbenkomponente (1501) der Spitze der Zuführvorrichtung übertragen wird, um die Hydraulikbetätigungskolbenkomponente (1501), die die Spitzenhülse (1503) distal verschiebt, mit Antriebskraft zu beaufschlagen,
wobei die Spitze eine hintere Hülse (1502) mit einer abgestuften Gestalt umfasst, die bezüglich des Kolbens verschiebbar zurückziehbar ist.

2. Zuführsystem nach Anspruch 1, wobei die Zuführvorrichtung dazu ausgestaltet ist, den Kolben distal zu verschieben oder den Kolben in einem Schraubengewinde zu drehen und sein distales Verschieben zu veranlassen.

3. Zuführsystem nach einem der Ansprüche 1 oder 2, wobei der Klappenstent selbstexpandierbar ist.

4. Zuführsystem nach einem der Ansprüche 1 bis 3, wobei die Zuführvorrichtung dazu ausgestaltet ist, einen minimalinvasiven chirurgischen Ansatz zu gestatten, bei dem eine Aortenklappenersatzintervention an einem schlagenden Herz durchgeführt wird, ohne dass eine geöffnete Brusthöhle und eine Herz-Lungen-Maschine notwendig wären.

5. Zuführsystem nach einem der Ansprüche 1 bis 4, wobei der Kardialklappenstent ein Klappenstent zum Ersatz einer Aortenklappe ist.

6. Zuführsystem nach einem der Ansprüche 1 bis 5, wobei der Kardialklappenstent ein Klappenstent zum Ersatz einer Mitralklappe ist.

7. Zuführsystem nach einem der Ansprüche 1 bis 6, wobei das Zuführsystem für transapikale oder für transvaskuläre Zuführung des Klappenstents ausgestaltet ist.

## Revendications

1. Système de pose de stent à valvule cardiaque pour la pose d'un stent à valvule cardiaque au niveau du coeur, comprenant un stent à valvule cardiaque et un dispositif de pose de stent à valvule cardiaque,
dans lequel le dispositif de pose comprend une partie terminale et la partie terminale comprend une gaine de partie terminale (1503) et un composant formant piston d'actionneur hydraulique (1501) qui est accouplé à la gaine de partie terminale (1503),
dans lequel le dispositif de pose est conçu pour utiliser une action hydraulique qui est transmise au composant formant piston d'actionneur hydraulique (1501) de la partie terminale du dispositif de pose par le biais d'un conduit étanche aux fluides pour appliquer une force d'entraînement au composant formant piston d'actionneur hydraulique (1501) qui déplace dans le sens distal la gaine de partie terminale (1503),
dans lequel la partie terminale comprend une gaine arrière (1502) présentant une forme à gradin qui est rétractable de manière coulissante relativement au piston.

2. Système de pose selon la revendication 1, dans lequel le dispositif de pose est conçu pour faire coulisser le piston dans le sens distal ou pour appliquer une rotation au piston dans un filetage de façon à provoquer son déplacement distal.

3. Système de pose selon l'une ou l'autre des revendications 1 et 2, dans lequel le stent à valvule est autoexpansible.

4. Système de pose selon l'une quelconque des revendications 1 à 3, dans lequel le dispositif de pose est conçu pour permettre une approche chirurgicale mini-invasive, une intervention chirurgicale de remplacement de valvule étant ainsi réalisée sur un coeur battant sans qu'une thoracotomie ou un pontage cardio-pulmonaire soit nécessaire.

5. Système de pose selon l'une quelconque des revendications 1 à 4, dans lequel le stent à valvule cardiaque est un stent à valvule pour le remplacement d'une valvule aortique.

6. Système de pose selon l'une quelconque des revendications 1 à 5, dans lequel le stent à valvule cardiaque est un stent à valvule pour le remplacement d'une valvule mitrale.

7. Système de pose selon l'une quelconque des revendications 1 à 6, le système de pose étant conçu pour une pose transapicale ou transvasculaire du stent à valvule.
